# EUROPEAN PATENT SPECIFICATION

(11) **EP 4 606 712 B1**
(45) Date of publication and mention of the grant of the patent: **03.06.2026**
(21) Application number: 25158806.7
(22) Date of filing: 19.02.2025
(51) Int. Cl.: B65B 5/04, B65B 31/02, B65B 31/04, B65B 55/10, B65B 55/14, B65B 57/16, A61L 2/14, A61L 2/24

(54) **A TREATMENT AND PACKAGING METHOD OF A REUTILISABLE INVASIVE MEDICAL DEVICE, A HEAT-SEALED UNIT COMPRISING A REUTILISABLE INVASIVE MEDICAL DEVICE AND A TREATMENT AND PACKAGING APPARATUS OF A REUTILISABLE INVASIVE MEDICAL DEVICE**
VERFAHREN ZUR BEHANDLUNG UND VERPACKUNG EINES WIEDERVERWENDBAREN INVASIVEN MEDIZINISCHEN GERÄTS, EINE WÄRMEVERSIEGELTE EINHEIT, DIE EIN WIEDERVERWENDBARES INVASIVES MEDIZINISCHES GERÄT ENTHÄLT, UND EINE VORRICHTUNG ZUR BEHANDLUNG UND VERPACKUNG EINES WIEDERVERWENDBAREN INVASIVEN MEDIZINISCHEN GERÄTS
PROCEDE DE TRAITEMENT ET D'EMBALLAGE D'UN DISPOSITIF MEDICAL INVASIF REUTILISABLE, UNITE THERMOSOUDEE COMPRENANT UN DISPOSITIF MEDICAL INVASIF REUTILISABLE ET APPAREIL DE TRAITEMENT ET D'EMBALLAGE D'UN DISPOSITIF MEDICAL INVASIF REUTILISABLE

(30) Priority: 21.02.2024 IT 202400003697
(43) Date of publication of application: 27.08.2025
(73) Proprietor: Medical Devices Group S.r.l., 48123 Mezzano (RA) (IT)
(72) Inventor: ZANIBONI, Benedetta, 48124 Ravenna (IT)
(74) Representative: Dall'Olio, Christian

(56) References cited:
- EP-A1- 4 309 680
- FR-A1- 2 821 557
- JP-A- 2001 309 966
- US-A1- 2011 139 650
- US-A1- 2017 049 916
- US-A1- 2017 274 108
- US-B2- 10 351 305

## Description

### Field of the invention

The invention relates to the sector of aseptic conservation of a reutilisable invasive medical device, comprising: a body; a plurality of internal channels, at least partially housed in the body; and a pressure-balance valve; which is arranged at the body and which is configurable in an open configuration so as to place the inside of the body and an environment (AA) outside the body in communication; and, henceforth, indicated for the sake of simplicity with the term "reutilisable invasive medical devices" or with the term "medical device". In particular, the invention relates to a treatment, packaging and storage method of the medical device, of the units that comprise the medical device and the apparatus and methods of packaging of the medical device.

### Description of the prior art

Among the reutilisable invasive medical devices, which comprise a plurality of internal channels, are included: endoscopes, laparoscopes, thoracoscopes, cystoscopes, ureterorenoscope, nephroscopes, arthroscopes, gastrocopes, colonoscopes, etc. These medical devices, being reutilisable, must be subjected to disinfection and drying before any subsequent use. When the reutilisation is not done immediately following disinfection, the devices must be adequately conserved to avoid a successive contamination.

Various types of storage devices of the disinfected and dried medical devices have been designed and, with the aim of reducing storage costs, bags made of single-use plastic material have been used for packaging and conserving them while awaiting a subsequent use.

A first known method of packaging and optional treatment of the above-mentioned medical devices used: a drying and treatment unit of a medical device; a printer which is electrically connected to the drying and treatment unit; a plurality of hydraulic tubes, which are hydraulically connectable, at a first end, to the drying and treatment unit and, at a second end, to the channels of the medical device; a bag made of a food-grade plastic material provided with an adhesive flap. The adhesive flap is arranged at the opening of the bag, and is foldable to close the opening; and a further hydraulic tube having a first end able to perforate the bag and a second end connectable to the drying and treatment unit. The drying and treatment unit is able to dispense: a drying gas (which can be medical grade nitrogen or medical grade air), or a gas containing ozone, generated by an electrical discharge in the flow of drying gas. Once washed and disinfected, the medical device comprising the plurality of internal channels connects hydraulically, via the plurality of hydraulic tubes, the plurality of channels to the drying and treatment unit. After this, it supplies, into the plurality of channels, the drying gas at a low flow-rate (1-20 I/min) at a temperature comprised between 10 and 30°C for a time interval comprised between 10 and 60 seconds. This means that, in the plurality of channels, there is a laminar flow having a Reynolds number that is lower than 2300 to avoid fragmentation of the flow of drying gas. Thereafter, the drying gas is supplied into the plurality of channels or, alternatively, the gas containing ozone at a flow-rate (20-100 I/min), at a temperature comprised between 30 and 60°C for a time interval comprised between 60 and 140 seconds, creating, in the plurality of channels, a turbulent flow having a Reynolds number of higher than 2300. At this point, it disconnects, hydraulically and manually, the medical device from the drying unit and inserts it manually into the bag, the terminal opening of the bag is closed manually by folding the adhesive flap to close the opening of the bag and obtaining a bag/medical device unit. The second end of the further hydraulic tube is then hydraulically connected to the drying and treatment unit, the bag of the bag/medical device unit is perforated with the second end of the further hydraulic tube, then the gas containing ozone is supplied for a time period comprised between 3 and 10 seconds. The second end of the further hydraulic tube is extracted from unit and the hole is closed with an adhesive label printed by the printer and bearing the data relative to the treatment carried out. When the gas containing ozone is used, the aseptic conservation of the medical device is possible for 72 hours, which means that within 72 hours, the microbial load present on the medical device remains unaltered. Note that, between the extraction of the second end of the further hydraulic tube from the bag/medical device unit and the closure of the hole by means of the label, the inside of the unit is in contact with the environment outside the bag/medical device unit. Besides this, the adhesive surface of the label, before being applied on the hole, is located in the environment (AA) outside from the bag/medical device unit. Therefore, once the label has been applied to the bag/medical device unit at the position of the through-hole, a part of the adhesive surface of the label is in contact with the inside of the bag/medical device unit. Further, many steps are manual. All the listed factors can cause a contamination of the medical device of the bag/medical device unit.

Further, the closure of the opening of the bag with the adhesive flap and the adhesive label do not guarantee a closure of the bag that is perfectly hermetic and are manual operations that can suffer from the presence of folds in the bag/medical device unit, as well as human error. Additionally, it is not possible to simply verify whether the bag of the bag/medical device unit is still intact during the conservation thereof.

A vacuum packaging and conservation system of a reutilisable invasive medical device is known, comprising a plurality of internal channels. The system comprises a sealable internal chamber suitable for receiving the medical device. The internal chamber comprises a tray and a cover and an evacuating single-acting valve arranged on the cover which allows evacuation only of gas from the internal chamber. The system further comprises a sealable external chamber constituted by a flexible bag, having a closable terminal opening comprising a pressure zip and an evacuation valve for connection to a suction device for evacuation of the external chamber. The internal chamber is insertable in the external chamber through the reclosable opening. The vacuum packaging and conservation method of a reutilisable invasive medical device uses the vacuum packaging and conservation system. It comprises following steps: inserting the reutilisable invasive medical device, washed, dried and disinfected, into the internal chamber; atomising, in the internal chamber, a solution of hydrogen peroxide; sealing the internal chamber; inserting the internal chamber in the external chamber; sealing the external chamber; connecting the evacuation valve of the external chamber to a suction device; activating the suction device so as to evacuate the external chamber, causing tof the single-acting valve of the internal chamber to open, so as also to evacuate the internal chamber; deactivating the suction device by closing the single-acting valve and the evacuation valve. It is clear that this vacuum storage and conservation method leads to handling the reutilisable invasive medical device, already disinfected, with the purpose of inserting it into the first chamber, and that the closure using a pressure zip of the bag does not ensure a hermetic seal of the second chamber over a period of time. The solution of hydrogen peroxide, when the medical device is reutilised in short times can damage the body mucosa. Additionally, the solution of hydrogen peroxide is aqueous and the internal chamber is closed, and this might facilitate bacterial proliferation. Therefore, this method too does not ensure a bacteriologically safe conservation of the invasive and re-utilisable medical devices. Further, it requires a first chamber that is relatively costly, given that it is for single use, and which, if reutilisable, must be carefully disinfected before subsequent re-use. US2017274108 discloses a treatment and packaging in a bag apparatus and method of a medical device in which no internal depression is created in the bag.

There thus emerges the need to conserve, in a biologically safe way, once washed, dried and disinfected and until the subsequent re-use, of the reutilisable invasive medical devices comprising: a body; a plurality of internal channels, at least partially housed in the body; and a pressure-balance valve; which is arranged at the body and which is configurable in an open configuration so as to place the inside of the body and an environment outside the body in communication.

It is observed that all the known methods store these medical devices, dried previously by an operator or by use of a device suitable for drying the medical devices.

### Disclosure of the Invention.

An object of the present invention is to enable conservation of the reutilisable invasive medical devices in a biologically safe way, with reutilisable invasive medical devices comprising a plurality of internal channels and which have been washed and disinfected, until subsequent use thereof, while guaranteeing that they are stored dry.

A further main object consists in providing and being able to verify, in a simple way, whether, during conservation, the medical device has been contaminated, or not.

A further main aim of the present invention is to provide a treatment and packaging method that is simple, reliable and has relatively modest costs with respect to the objectives which are set and which does not involve the need to use a containing element of the medical device that is relatively expensive and which requires disinfection in order to be reutilised. Further, the invention has the aim of not handling and contaminating a disinfected medical device when inserting it in a containing element.

The invention further includes utilising, for these aims and objectives, an economical single-use container which guarantees a perfect hermetic seal.

A secondary aim of the present invention consists in guaranteeing that the medical devices are conserved dry in order to prevent the possibility of bacterial proliferation due to the presence of water.

The aforementioned aims and main objects have been achieved with a treatment and packaging method of a reutilisable invasive medical device (comprising a plurality of internal channels), a heat-sealed unit comprising a reutilisable invasive medical device and a treatment and packaging apparatus of a reutilisable invasive medical device according, respectively, to the following independent claims.

According to the invention, the medical device can be treated while it is arranged internally of the bag of the heat-sealable unit, which will subsequently be heat-sealed without the treated medical device being handled or coming into contact with the environment outside of the heat-sealable unit and in particular the environment outside the disinfection and packaging apparatus. Consequently, the treatment and packaging apparatus is able to carry out, in a totally automatic way, steps G) H), and I) of the treatment and packaging method according to the invention.

Additionally, the sealing of the bag of the heat-sealed unit guarantees a perfect hermetic seal of the heat-sealed unit which is reliable over time, allowing aseptic conservation of the medical device contained therein. Besides this, as the heat-sealing takes place when, internally of the heat-sealable unit, a fourth predetermined depression is present, it is possible to visually verify, with certainty, whether the heat-sealed unit remains unaltered during conservation, or whether it is damaged. In fact, in a case in which the relative heat-sealed bag is damaged, it will be possible to verify visually that the heat-sealed unit no longer has a depression internally thereof. Therefore, according to the invention it is possible to verify in a simple way whether, during the conservation of the heat-sealed unit that comprises the treated medical device, the seal remains total during conservation. Consequently, it is easy to confirm whether the medical device of the heat-sealed unit has been (or not) contaminated during the conservation thereof.

Note that the invention has the significant advantage of using, as a containing element of the medical device, a sterile single-use container, which is a simple and economical sterile single-use heat-sealable bag.

The applicant has verified that the heat-sealed unit according to the invention can be stored for 800 hours, without the occurrence of a bacterial proliferation. Consequently, according to the invention, it is possible to conserve the medical device aseptically for 800 hours.

The term "depression", is, obviously, taken toi mean a barometric depression, which is none other than a pressure that is lower than standard atmospheric pressure, indicating negative Pa with respect to standard atmospheric pressure. This is defined as the pressure exerted at sea level by a column of mercury 760 mm high and equal to 101,325 Pa. Therefore, for example, a depression of -10,000 Pa corresponds to a pressure of 91,325 Pa.

Cold plasma is a gas plasma that is highly reactive at low temperature. It can be generated in atmospheric conditions (101,325 P at about 25 °C) by a discharge a high voltage (3000 - 4000 V) between two metal electrodes.

### Brief description of the drawings

Specific embodiments of the invention will be described in the following part of the present description, according to what is set down in the claims and with the aid of the accompanying tables of drawings, in which:
figure 1 is a schematic and perspective view of a first embodiment of the apparatus according to the invention in a closed configuration:
figure 2 is a further schematic and perspective view of the apparatus of figure 1 in the same configuration;
figure 3 is a schematic view from above of a second embodiment of the treatment and packaging apparatus according to the invention in a configuration of partial opening:
   figure 4 is a further schematic view from above of the apparatus of figure 3 in a closed configuration:
figure 5 is a schematic front view and with some parts removed of the apparatus of figure 4 in the same configuration;
figure 6 is a schematic, perspective, lateral view, and with some parts removed, of the apparatus of figure 4 in the same configuration;
figure 7 is a further schematic perspective view, with some parts removed, of the apparatus of figure 4 in the same configuration;
figure 8 is a still further schematic and perspective view, with some parts removed, of the apparatus of figure 4 in the same configuration;
figure 9 is a further schematic, perspective view, with some parts removed, of the apparatus of figure 4 in an open configuration;
figure 10 is a hydraulic diagram of an apparatus according to the invention in a partially open configuration;
figure 11 is a further hydraulic diagram of the apparatus of figure 3 of an apparatus according to the invention;
figure 12 is a schematic and perspective view of a heat-sealable unit;
figure 13 is a schematic and perspective view of the heat-sealable unit of figure 12 and of the supply endof the supply device comprised in the treatment and packaging apparatus according to the invention;
figure 14 is a schematic, perspective and partial view of the heat-sealable unit of figure 12 coupled to the supply end of the supply device of figure 13;
figure 15 is a schematic perspective view of the heat-sealed unit according to the invention coupled to the supply end of the supply device of figure 13; and figure 16 is a schematic perspective view of the heat-sealed unit (8) of figure 15.

### Description of preferred embodiments.

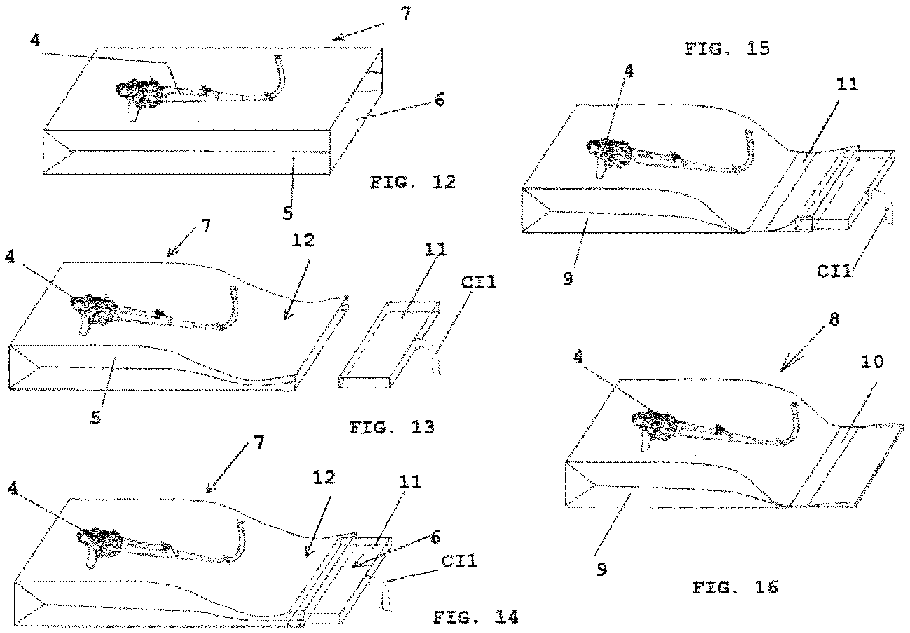

Note that in figures 13-16, for the sake of clarity, dotted lines are used for those elements that are not visible from outside relative to the coupling of the heat-sealable unit or the heat-sealed unit and of the supply end of the supply device while the medical device contained in the unit has not been represented with a dotted line because the bag is transparent.

With reference to the figures of the drawings, reference numeral (1) a treatment and packaging apparatus of a reutilisable invasive medical device according to the invention.

The characteristics of the invention will be described in the following in which some preferred but not exclusive embodiments thereofare described, embodiments of actuation/realisation of the treatment and packaging method of a reutilisable invasive medical device, of the heat-sealed unit (8) comprising a reutilisable invasive medical device and of the treatment and packaging apparatus (1) of a reutilisable invasive medical device.

The treatment and packaging method of a reutilisable invasive medical device according to the invention includes that the invasive medical device is reutilisable and comprises a plurality of internal channels.

The treatment and packaging method comprises, in the following order, following steps:
A) predisposing at least an air source which is a source of medical grade air (otherwise known as sterile air);
B) predisposing a cold plasma source;
C) predisposing a medical device (4): which is a reutilisable invasive medical device; which comprises: a body; a plurality of internal channels, at least partially housed in the body; and a pressure-balance valve (not illustrated);

which is arranged at the body and which is configurable in an open configuration so as to place the inside of the body and an environment (AA) outside the body in communication;
wherein the medical device (4) is:
   - in a substep C1) of the predisposing step C): clean; disinfected and dry;
   - in a substep C2) of the predisposing step C), which is alternative to substep C1): clean; disinfected and wet; and wherein substep C2) of the predisposing step C) comprises at least partially removing the water from the clean, disinfected and wet medical device (4) using medical grade and compressed air; D) predisposing a bag (5) which is at least of a grade for food use (i.e. which is at least suitable for conserving foodstuffs internally thereof): which is sterile (therefore suitable for conserving internally thereof a medical device (4)); which is made of a heat-sealable plastic material; which comprises a terminal opening (6); which is able to house internally thereof the medical device (4) predisposed in the predisposing step C) by inserting the medical device through the terminal opening (6);
E) activating the pressure-balance valve to open so as to place the inside of the body of the medical device (4) and an environment (AA) outside of the body in communication, thus obtaining a balanced medical device (4) wherein the inside of the body is at a same pressure as the environment (AA) outside the body;
F) inserting the balanced medical device (4) obtained in the activating step E) internally of the bag arranged in the predisposing step D), obtaining a heat-sealable unit (7) (see figure 12) which comprises: the bag (5) and the balanced medical device (4) arranged internally of the bag (5);
G) carrying out at least a treatment cycle, optionally at a predetermined temperature comprised between 20°C and 60°C (preferably between 25°C and 45 °C), wherein each at least a treatment cycle comprises substeps as follow:
   G1) obtaining, internally of the heat-sealable unit (7), a first predetermined depression;
   G2) supplying, to inside the heat-sealable unit (7), a treatment gas comprising (and, preferably, constituted by): cold plasma; and medical grade air, maintaining, inside the heat-sealable unit (7), a second predetermined depression (which is preferably lower than the first depression) for a predetermined time interval,
   H) obtaining, internally of the heat-sealable unit (7), a third predetermined depression;
   I) on conclusion of the obtaining step H), obtaining, internally of the heat-sealable unit (7), a fourth predetermined depression which is lower: than the first predetermined depression; than the second predetermined depression; and than the third predetermined depression; and heat-sealing the bag of the heat-sealable unit (7) obtaining a heat-sealed unit (8) (see figures 15 and 16) comprising: a heat-sealed bag (9), which is hermetically closed by at least a heat-sealing; and the balanced reutilisable medical device (4), arranged internally of the heat-sealed bag (9), wherein, the fourth predetermined depression is present internally of the heat-sealed bag (9). Obviously, as the heat-sealed bag (9) is hermetically closed by at least a heat-sealing and is in depression, the bag comprises no opening. This is obtainable when the bag predisposed in the predisposing step D) is a normal bag comprising a single opening, which is not present in the heat-sealed bag (9), by virtue of step I) of obtaining the fourth depression and heat-sealing.

Obviously, substep C2) of the predisposing step C) comprises passage of medical grade and compressed air in the plurality of internal channels;

The treatment and packaging method enables obtaining a heat-sealed unit (8) comprising:
- a heat-sealed bag (9); which is made of a heat-sealable plastic material; and which is hermetically closed by at least a heat-sealing (10); and
- a medical device (4), which is a reutilisable invasive medical device; which comprises: a body; a plurality of internal channels at least partially housed in the body; and a pressure-balance valve (not illustrated), which is arranged at the body and which is activatable to open so as to place the inside of the body and an environment (AA) outside the body in communication, wherein the pressure-balance valve is activated to open; and wherein the medical device (4) is dry and is arranged internally of the heat-sealed bag (9), and wherein a predetermined depression is present internally of the heat-sealed bag (9). This depression is preferably comprised between -10,000 Pa (100 mbar) and - 100,000 Pa (-1000 mbar), advantageously between -8,000 Pa and -12,000 Pa, and more advantageously is about -10,000 Pa.

The heat-sealed bag is obtained starting from a heat-sealable bag which has been previously sterilisated.

When the pressure-balance valve is activated to open it is in a relative open configuration in which it allows hydraulic communication between the inside of the body of the medical device (4) and the environment outside the medical device (4).

Treatment and packaging methods are preferred according to the invention, in which step G) of carrying out the at least a treatment cycle is the following:
G) carrying out at least a treatment cycle, wherein, when substep C2) is included of the predisposing step C), the at least a treatment cycle is carried out at a predetermined temperature comprised between 20°C and 60°C (preferably between 25°C and 45°C more preferably between 35°C and 45°C), and wherein, when substep C1) of step C) of the predisposing step is included, the at least a treatment cycle is optionally carried out at the predetermined temperature, (which can preferably be comprised between 25°C and 45°C) and wherein each treatment cycle comprises substeps G1) and G2).

Note that, in that case, when substep C2) of step C) of the predisposing step is included, the treatment and packaging method enables packaging a medical device that is perfectly dry by virtue of the predetermined temperature and the fact that sub-steps G1) and G2) of step G) of carrying out at least a treatment cycle are carried out in a depression.

While when substep C1) of step C) of the predisposing step is included, by virtue of the fact that substeps G1) and G2) of step G) of carrying out at least a treatment cycle are carried out in a depression, this step is sufficient to maintain, during the at least a treatment cycle, the predisposed clean medical device clean, disinfected and dry. The clean, disinfected and dry medical device can be collected by an appropriate drying and storage cabinet.

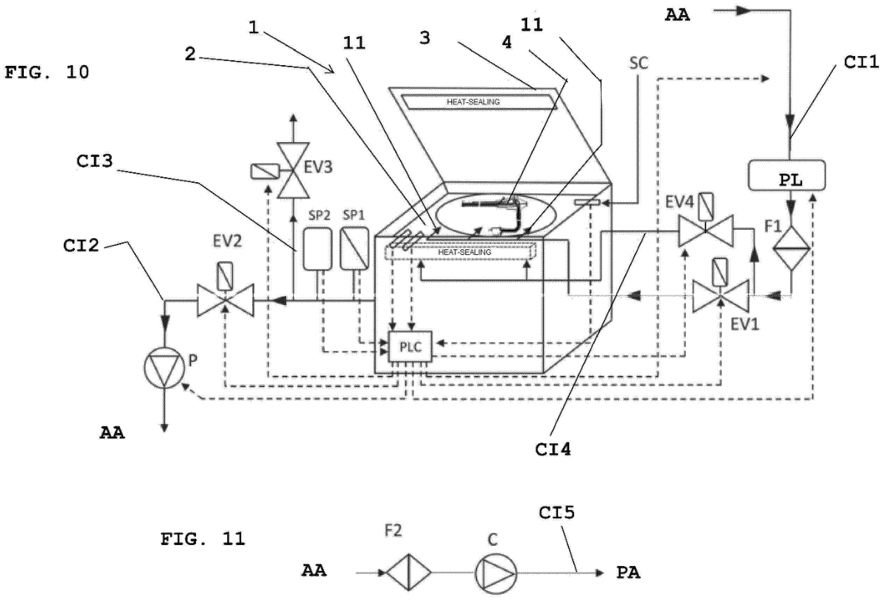

The treatment and packaging apparatus (1) of a reutilisable invasive medical device according to the invention enables actuating the treatment and packaging method. The treatment and packaging apparatus (1) comprises:
- a housing (2) comprising: an opening, a cover (3) for closing the opening; and a side; wherein the opening comprises a side, wherein the cover (3) comprises a side and wherein, with the cover (3) closing the opening, the housing (2) is closed, and the side of the opening and the side of the cover (3) are arranged at the side of the housing (2);
- a supply device: which is activatable to supply a gas internally of the housing (2) when the housing (2) is closed: the supply device having a terminal supply end (11), wherein the terminal supply end (11): is arranged in the housing (2); and projects from the side of the housing (2);
- a first heat-sealing element (TS1) and a second heat-sealing element (TS2) (see figure 10), wherein the first heat-sealing element (TS1) is arranged inside the housing (2) at the side of said housing (2); wherein the second heat-sealing element (TS2) is arranged internally of the housing (2) and is fixed to the cover (3) at the side of the cover (3); wherein, with the housing (2) closed, the first heat-sealing element (TS1) and the second heat-sealing element (TS2) are opposite one another; wherein at least one from between the first heat-sealing element (TS1) and the second heat-sealing element (TS2) is activatable to heat-seal (i.e. is heatable and, preferably, also movable) a heat-sealable plastic material, when the heat-sealable plastic material is interposed between, and in contact with, the second heat-sealing element (TS1) and the second heat-sealing element (TS2), which are opposite one another; and wherein the housing (2) is dimensioned so as to house a heat-sealable unit (7) (see figure 12) in an operating position, which heat-sealable unit (7) comprises: a bag (5), which is made of the heat-sealable plastic material and which comprises a terminal opening (6) and a sealing section (12) which is proximal to the terminal opening (6); and a medical device (4), which is a reutilisable invasive medical device (clean, disinfected and dry), which comprises a plurality of internal channels, and which is arranged internally of the bag; wherein, with the heat-sealable unit (7) in the operating position and the housing (2) closed, the bag of the heat-sealable unit (7) has: the terminal opening (6) arranged around, and in contact with, the terminal supply end (11) of the supply device, and the sealing section (12) which is arranged between the first heat-sealing element (TS1) and the second heat-sealing element (TS2), which are opposite one another, in order to enable a hermetic closing by heat-sealing of the bag (5);
- a production device (PL), which is hydraulically connectable to the supply device and which is activatable to produce cold plasma (and which, preferably, is a production device of cold plasma according to Italian patent IT 102020000019462);
- a suction device which is activatable so as to obtain, with the housing (2) closed, a depression internally of the housing (2)
- at least a pressure sensor (SP1, SP2); which is hydraulically connected to the housing (2); and which is activatable to measure a pressure in the housing (2) (especially when the cover (3) closes the housing (2)); and
- an electronic control and command device (PLC) (which can preferably be a programmable logic controller), which is electrically connected: to the supply device; to the production device (PL); to the suction device; to the at least a pressure sensor (SP1, SP2); and to the at least one from between the first heat-sealing element (TS1) and the second heat-sealing element (TS2) which is activatable; wherein the electronic control and command device (PLC) is configured to activate said at least a pressure sensor (SP1, SP2) and to monitor the pressure internally of the housing (2) by means of the at least an activated pressure sensor (SP1, SP2) and for, when the housing (2) is closed, activating the production device (PL) and carrying out the following treatment and packaging operation in order as follows:
   a) disinfecting by carrying out:
      a1) activating the suction device so that said at least a pressure sensor (SP1, SP2) measures a first predetermined depression;
      a2) connecting the production device (PL) to the first supply device, and activating the supply device to supply a treatment gas, comprising medical grade air and cold plasma, into the housing (2), maintaining, internally of the housing (2), a second depression for a predetermined time interval;
   b) activating the suction device so that said at least a pressure sensor (SP1, SP2) measures a third predetermined depression;
   c) activating the suction device so that said at least a pressure sensor (SP1, SP2) measures a fourth predetermined depression which is lower: than the first predetermined depression; than the second predetermined depression; and than the third predetermined depression;
   d) activating the at least one from between the first heat-sealing element (TS1) and the second heat-sealing element (TS2) which is activatable, maintaining the fourth predetermined depression; and
   f) deactivating the suction device, obtaining, in the housing (2), a pressure that is equal to the environment pressure (AA) outside of the treatment and packaging apparatus (1).

This enables the treatment and packaging apparatus (1), when the heat-sealable unit (7) is housed in the housing in the housing (2) in the operating position with the housing (2) closed, to carry out, in the following order, following operations:
- to aspirate the gases present inside the closed housing (2) up until obtaining therein a predetermined first depression with a consequent obtaining, inside the heat-sealable unit (7), housed in the closed housing (2), of the first depression;
- to supply the treatment grade gas to the heat-sealable unit (7) while the gases present internally of the closed housing (2) are aspirated, maintaining, in the closed housing (2), and therefore also in the heat-sealable unit (7), the second depression for the predetermined time interval;
- to aspirate the gases present inside the closed housing (2) up until obtaining therein a predetermined third depression with a consequent obtaining, inside the heat-sealable unit (7), housed in the closed housing (2), the first depression;
- to heat-seal the bag of the heat-sealable unit (7) while the at least a pressure sensor (SP1, SP2) measures the fourth predetermined depression, obtaining a heat-sealed unit (8) according to the invention.

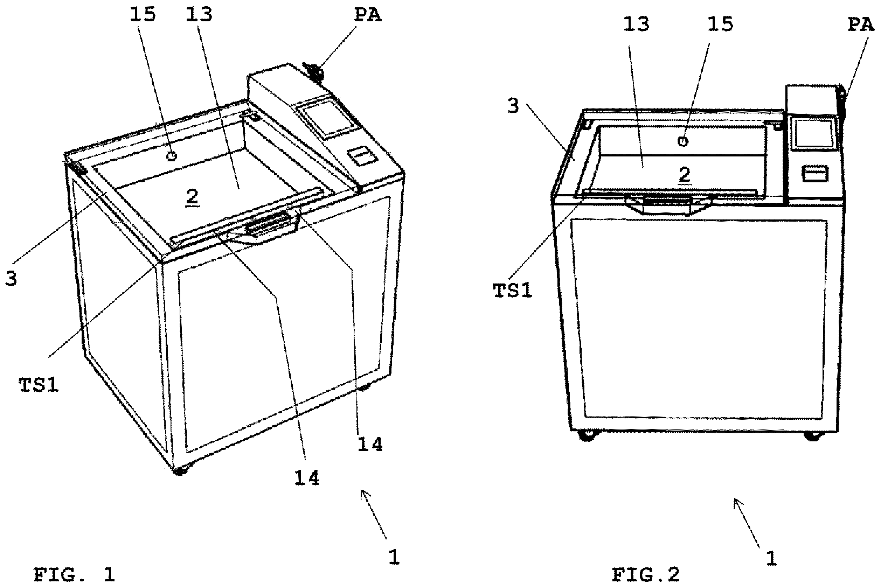

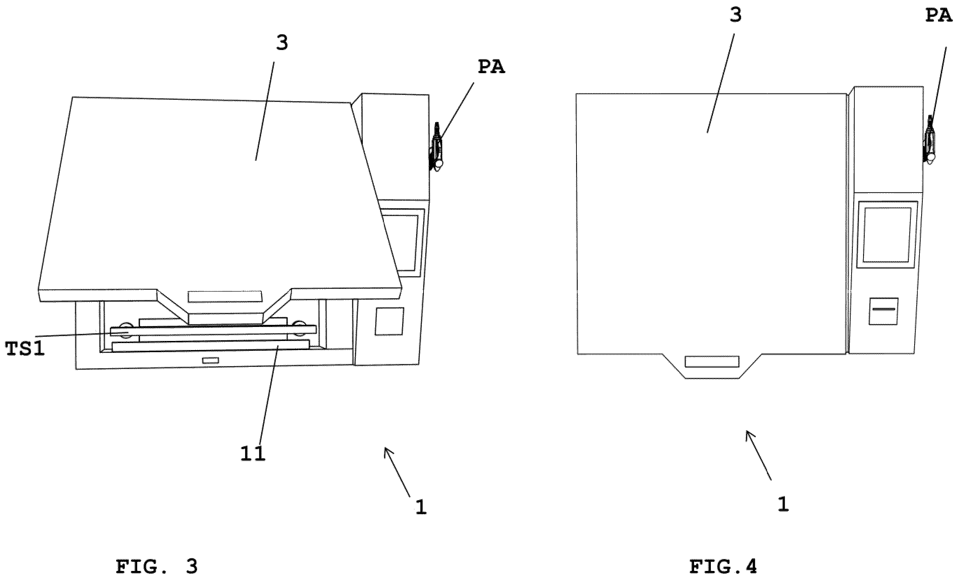

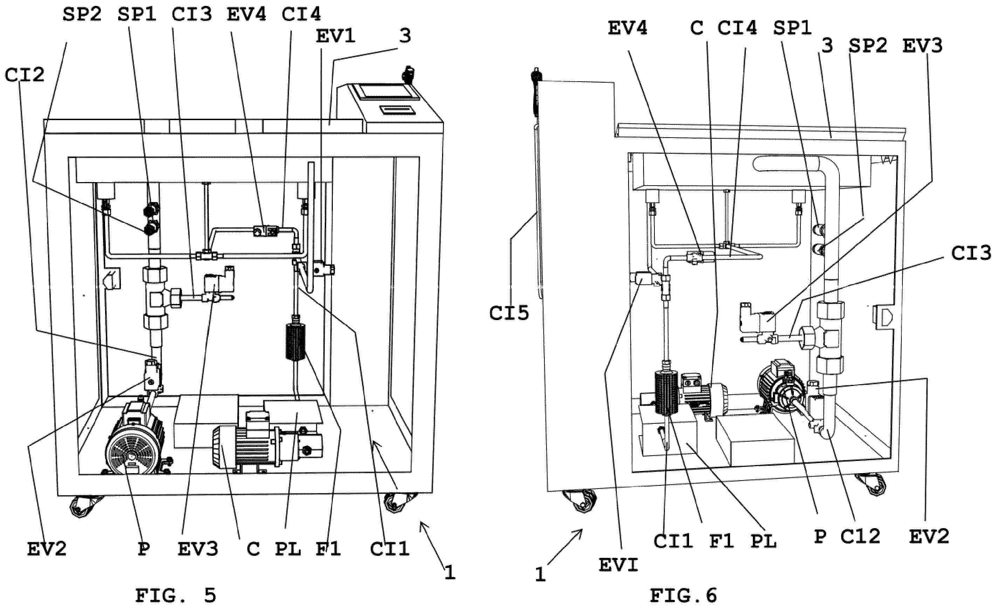

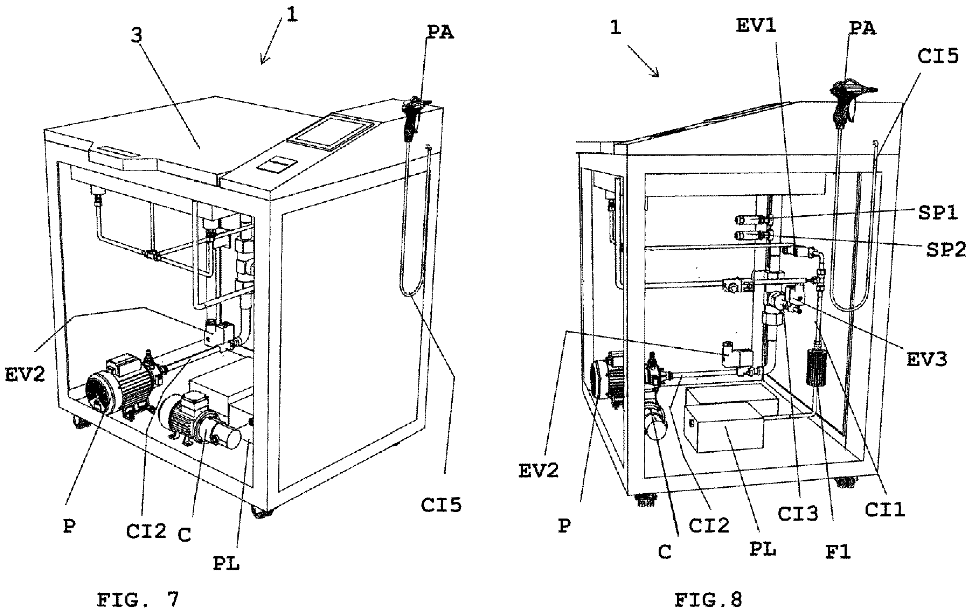

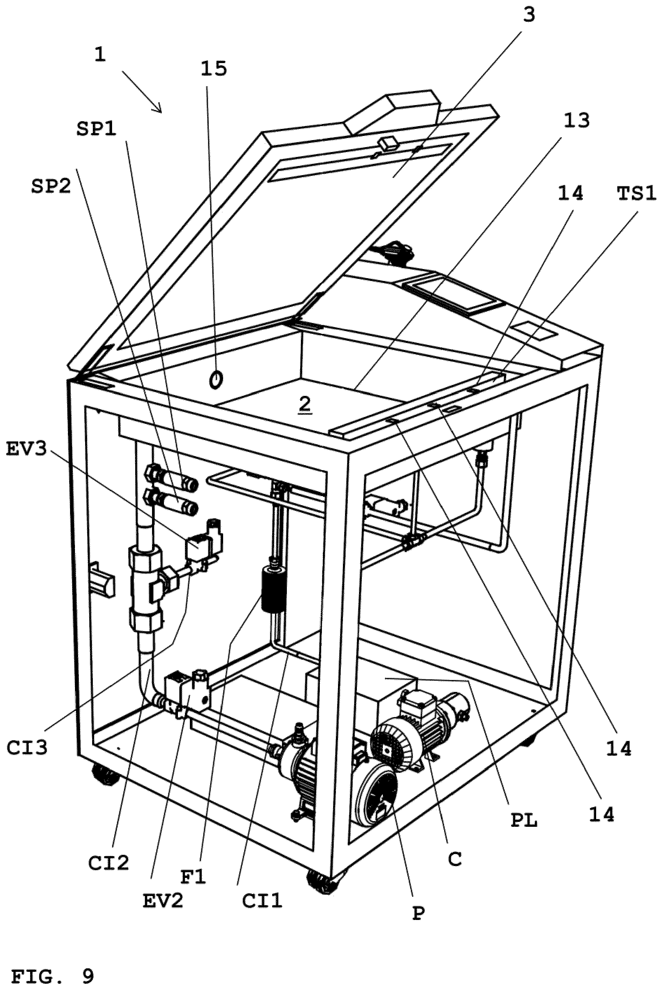

Note that in the embodiment of figures 1 and 2, the cover (3) is transparent, while in the embodiment of figures 3-9 it is not.

Preferably, both with respect to the treatment and packaging method and with respect to the treatment and packaging apparatus according to the invention, the second predetermined depression can be lower than the first predetermined depression, the third predetermined depression can be greater than the second predetermined depression. More preferably, the first predetermined depression and the third predetermined depression are comprised between -10,000 Pa (100 mbar) and -100,000 Pa (-1000 mbar) and the second predetermined depression can be comprised between -10,000 Pa (-100 mbar) and -25,000 Pa (-250 mbar). More advantageously the first predetermined depression, the third predetermined depression and optionally the fourth depression can be comprised between -25,000 Pa (250 mbar) and - 75,000 Pa (-750 mbar) and the second predetermined depression can be comprised between -0.001 Pa (-100 mbar) and -17,000 Pa (-175 mbar). The first predetermined depression and the third predetermined depression are preferably of about -30,000 Pa the fourth depression is comprised between - 10,000 Pa and -20,000 Pa more preferably comprised between -8,000 Pa and -12,000 Pa, and is advantageously about -10,000 Pa.

Therefore, in the heat-sealed unit (8) inside the heat-sealed bag, there is a predetermined depression preferably comprised between -10,000 Pa (-100 mbar) and -500 Pa (-5 mbar) preferably comprised between -10,000 Pa and - 25,000 Pa advantageously comprised between -10,000 Pa and -20,000 Pa and more advantageously of about -10,000 Pa.

The first and third depression can also be identical and, preferably, the fourth depression is lower than the second.

Note that in the treatment and packaging apparatus according to the invention, when the heat-sealable unit (7) is arranged in the operating position and the housing (2) closed, any depression internally of the housing (2), acts on the external walls of the heat-sealable unit (7), i.e. on the external walls of the relative bag (which, implicitly, is flexible) by compressing it to cause the gases contained therein to be evacuated, while enabling, at the same time, the supply of the gas into the heat-sealable unit (7) through the terminal supply end (11) of the supply device. During the compression of the walls of the heatformable unit, the gases contained therein are forced to exit through the terminal opening (6) of the bag through the space (which is in any case always present) between the terminal opening (6) of the bag and the terminal supply end (11).

The production device (PL) advantageously comprises (or is constituted by) a generator of a cold plasma, which, preferably, can be a generator of a cold plasma according to Italian patent IT102020000019462.

To further guarantee the hermetic seal of the heat-sealed unit (8), it is preferable, both in the treatment and packaging method and in the heat-sealed unit (8) for the at least a heat-sealing (10) to comprise a first heat-sealing and a second heat-sealing, which are arranged substantially parallel to one another.

For this purpose the at least one from between the first heat-sealing element (TS1) and the second heat-sealing element (TS2) which is activatable of the treatment and packaging apparatus (1), can comprise two heatable contact surfaces not joined to one another and, preferably, parallel.

A particularly advantageous embodiment of the treatment and packaging apparatus according to the invention is one wherein the housing (2) further comprises a bottom wall (13), which, when the cover (3) closes the housing, is opposite the cover (3), wherein the treatment and packaging apparatus (1) further comprises:
- a heating element (R) (preferably an electrical resistance): and which is activatable so as to be heated; and which is arranged externally of the housing, preferably at and in contact with the bottom wall (13);
- at least a first temperature sensor (ST1): which is arranged in the housing (2); and which is activatable to measure the temperature internally of the housing (2);
- at least a second temperature sensor (ST2); which is arranged proximally to the heating element (R); and which is activatable to measure the temperature of the heating element (R);
wherein the electronic control and command device (PLC) is further electrically connected: to the heating element (R); to the at least a first temperature sensor (ST1); to the at least a second temperature sensor (ST2); and wherein the electronic control and command device (PLC) is configured for, before carrying out the operations of treatment and packaging a)-f) or contemporaneously with the operation a1) of activating the suction device, activating: the heating element (R); the at least a first temperature sensor; and the at least a second temperature sensor; maintaining the housing at a predetermined temperature of between 20°C and 60°C (preferably between 25°C and 45 °C), monitoring the temperature internal of the housing (2) by means of the at least a first temperature sensor (ST1) and monitoring the temperature at the heating element (R) by means of the at least a second temperature sensor (ST1). This enables guaranteeing the complete elimination of water from the medical device, in particular from the relative plurality of internal channels and therefore, meaning that the heat-sealed unit does not contain water, eliminating the possibility of bacterial proliferation due to the presence of water.

Taking account of the physical factor relative to the first depression exerted on a heat-sealable unit, and owing to the heating element (R), arranged beneath the housing and not in contact with the heat-sealable unit, the certainty is given of total evaporation of the moisture or the water which might be present, by error, on or inside the reutilisable invasive medical devices, which comprise a plurality of internal channels, and in particular any moisture inside the plurality of internal channels. This ensures aseptic conservation of the medical devices.

In an embodiment of the treatment and packaging apparatus (1) the heating element (R) is arranged at and in contact with the bottom wall (13). In this case, the heating element can preferably be an electrical resistance (R) and preferably an adhesive electrical resistance, as it guarantees a more effective heating of the housing (2).

When the heating element (R) is present, it is preferable for the treatment and conservation apparatus to comprise a support element housable in the housing for separating the heat-sealable unit (7), arranged in the operating position, from the bottom wall (13). A preferred embodiment of the treatment and packaging method according to the invention is wherein in the step G) of carrying out at least a treatment cycle, a plurality of disinfection cycles is carried out. The plurality of disinfection cycles is advantageously comprised between 2 and 6. In the case of a plurality of disinfection cycles, the predetermined time interval can preferably be comprised between 1 and 300 seconds and then preferably progressively in the following order: between 50 and 300 seconds, between 100 and 300 seconds, between 150 and 300 seconds, between 200 and 300 seconds, and between 250 and 300 seconds.

With particular reference to figures 3, 9 and 10, the treatment and packaging apparatus (1) according to the invention can comprise an electronic closing device (20) activatable to close and open. In this case, the electronic control and command device (PLC) is further electrically connected to the electronic closing device (20), and the electronic control and command device (PLC) is configured to carry out the operations of treatment and packaging a)-f) only when the electronic closing device (20) is activated to close and, preferably, in order to activate the opening of the electronic closing device only on termination of the operation f) of deactivation of the suction device.

With particular reference to figure 10, a particularly preferred embodiment of the treatment and packaging apparatus (1) according to the invention can comprise:
- a first hydraulic duct (CI1): having a first end in hydraulic communication with the environment (AA) outside the treatment and packaging apparatus (1) and a second end, wherein the production device (PL) is hydraulically connectable to the first hydraulic duct (CI1) at a first connecting point, which is arranged along the first hydraulic duct (C11) between the first end of the first hydraulic duct (CI1) and the second end of the first hydraulic duct (CI1);
- a filter (F1): which is a suitable filter for filtering viruses and bacteria from the air (which preferably has pores of dimensions of smaller than 0.005 µm and more preferably smaller than 0.003 µm); and which is arranged along the first hydraulic duct (CI1) between the first connecting point and the second end of the first hydraulic duct (CI1);
- a first hydraulic intercept device (EV1) (preferably a solenoid) which is arranged along the first hydraulic duct (C11) between the filter (F1) and the second end of the first hydraulic duct (CI1);
- a second hydraulic duct (CI2) which has a first end (15) (see figures 1, 2 and 9) which is in hydraulic communication with the housing (2) (preferably on the opposite side to the side of the housing (2)) and a second end which is in hydraulic communication with the environment (AA) outside the treatment and packaging apparatus (1);
- a second hydraulic intercept device (EV2) (preferably a solenoid) which is arranged along the second hydraulic duct (cI2) between the first end (15) of the second hydraulic duct (CI2) and the second end of the second hydraulic duct;
- a suction element (P) (preferably a gas aspiration pump) which is arranged along the second hydraulic duct (CI2) between the second end of the second hydraulic duct (cI2) and the second hydraulic intercept device (EV2);
- a third hydraulic duct (CI3) which: has a first end in hydraulic communication with a second connecting point which is arranged along the second hydraulic duct (cI2) between the first end of the second duct and the second hydraulic intercept device (EV2); and a second end which is in hydraulic communication with the environment (AA) outside the treatment and packaging apparatus (1);
- a third hydraulic intercept device (EV3) (preferably a solenoid) arranged along the third hydraulic duct between the first end of the third hydraulic duct (cI3) and the second end of the third hydraulic duct (CI3);

wherein said at least a pressure sensor (SP1, SP2) is arranged in a position selected from between: a first position internally of the housing (2); a second position arranged along the second hydraulic duct between the first end (15) of the second hydraulic duct (CI2) and the second hydraulic intercept device (EV2); a third position arranged along the third hydraulic duct (cI3) between the first end of the third hydraulic duct (CI3) and the third hydraulic intercept device (EV3); and a fourth position arranged along the first hydraulic duct (C11) between the first hydraulic intercept device (EV1) and the second end of the first hydraulic duct;
wherein the suction device comprises: a portion of the first hydraulic duct (C11) arranged between the first hydraulic intercept device (EV1) and the second end of the first hydraulic duct, the first hydraulic intercept device (EV1), the second hydraulic duct (CI2); the second hydraulic intercept device (EV2); the suction element (P); the third hydraulic duct (CI3); and the third hydraulic intercept device (EV3);
wherein the supply device comprises: the suction device; the filter (F1); a portion of the first duct comprised between the first hydraulic intercept device (EV1) and the first end of the first hydraulic duct (C11) and wherein the second end of the first hydraulic duct (C11) constitutes the terminal supply end (11) of the supply device;
wherein the second depression is lower than the first predetermined depression, wherein the third predetermined depression is greater than the second predetermined depression; and wherein the fourth depression is lower than the third depression; wherein the electronic control and command device (PLC) is further electrically connected: to the first hydraulic intercept device (EV1); to the second hydraulic intercept device (EV2); to the third hydraulic intercept device (EV3), to the suction element (P); and wherein the electronic control and command device (PLC) is configured in order to carry out following operations (of treatment and packaging operation a-c), in the following order:
   a) disinfecting by carrying out:
      a1) activating the suction device, so that said at least a pressure sensor (SP1, SP2) measures the first predetermined depression: activating the first hydraulic intercept device (EV1) to close; activating the third hydraulic intercept device (EV3) to close; activating the second hydraulic intercept device (EV2) to open; and activating the suction element (P);
      a2) hydraulically connecting the production device (PL) to the first supply device, and activating the supply device to supply a treatment gas, comprising medical grade air and cold plasma, into the housing (2), maintaining, inside the housing (2), the second depression for a predetermined time interval, activating the first hydraulic intercept device to open (EV1);
   b) activating the suction device, so that said at least a pressure sensor (SP1, SP2) measures the third predetermined depression, activating the first hydraulic intercept device to close (EV1);
   c) activating the suction device so that said at least a pressure sensor (SP1, SP2) measures the fourth predetermined depression, activating the first hydraulic intercept device to open (EV1); and
   d) activating the at least one from between the first heat-sealing element (TS1) and the second heat-sealing element (TS2) which is activatable, maintaining the fourth predetermined depression inside the housing (2); and
   f) deactivating the suction device, obtaining, in the housing (2), a pressure that is equal to the pressure of environment (AA) outside the treatment and packaging apparatus (1), activating the third hydraulic intercept device to open (EV3) and optionally deactivating the suction element (P).

The supply device can preferably also comprise a compressor (not illustrated): which is in hydraulic communication with the first hydraulic duct (C11) at a first connecting point, which is arranged along the first hydraulic duct (C11) between the first end of the first hydraulic duct (C11) and the production device (PL); in which the compressor is electrically connected to the electronic control and command device (PLC) and is activatable to convey air along the first hydraulic duct (CI1), from the first end of the first hydraulic duct (C11) to the second end of the first hydraulic duct (CI1). In this case, the electronic control and command device (PLC) is configured to carry out the following operation a2) of hydraulic connection of the production device (PL) to the first supply device, and activation of the supply device to supply a treatment gas, comprising medical grade air and cold plasma, into the housing (2), maintaining, inside the housing (2), the second depression for a predetermined time interval, activating the first hydraulic intercept device to open (EV1) and activating the compressor.

According to a preferred embodiment of the heat-sealed unit (8) of the invention, further comprised is a pressure-balance cap (not illustrated) which is engageable with the pressure-balance valve to activate the valve to open, wherein the pressure-balance cap is engaged with the pressure-balance valve; and an RFID label (also known as a RFID tag" or "transponder") fixed to the pressure-balance cap, preferably fixed to an external wall thereof. An embodiment of the treatment and packaging apparatus (1) according to the invention which, consequently, is advantageous, can comprise a RFID reading device (not illustrated), in which the RFID reading device is arranged in the housing (2), is electrically connected to the electronic control and command device (PLC) and is activatable to read a RFID label. This enables reading the RFID label fixed to the pressure-balance cap which cap is engaged with the pressure-balance valve of a balanced medical device (4) comprised in the heat-sealable unit (7) when the unit is arranged in the operating position. This enables verifying whether the pressure-balance cap is present in the heat-sealable unit (7) and, in particular whether it is engaged with the pressure-balance valve. In this way, any eventual issues relative to poor pressure balance of the medical device (4) subjected to a depression are resolved. The electronic control and command device (PLC) can preferably be configured, before carrying out the treatment and packaging operations, to activate the RFID reading device and, only following confirmation, by the RFID reading device, that a RFID label reading has taken place, to carry out the treatment and packaging operations. In this way, it is possible to verify the presence of the pressure-balance cap by reading the RFID label fixed to the pressure-balance cap of the heat-sealable unit (7) in the operating position.

The RFID reading device is advantageously arranged in the housing (2) on the opposite side to the cover (3) of the housing (2), i.e. on the bottom wall (13) of the housing (2).

With the purpose of using a pressure difference to activate the at least one from between the first heat-sealing element (TS1) and the second heat-sealing element (TS2) which is activatable, it is preferable for the treatment and packaging apparatus (1) according to the invention to further comprise:
- a fourth hydraulic duct (CI4) in turn comprising: a first end which is in hydraulic connection with the first hydraulic duct (CI1) at a third connecting point, arranged along the first hydraulic duct (CI1), between the filter (F1) and the first hydraulic intercept device (EV1); and a second end which is connected to at least one from between the first heat-sealing element (TS1) and the second heat-sealing element (TS2) which is activatable to hydraulically move the latter towards the remaining element between the first heat-sealing element (TS1) and the second heat-sealing element (TS2);
- a fourth hydraulic intercept device (EV4) (preferably a solenoid) arranged along the fourth hydraulic duct (CI4) between the first end of the fourth hydraulic duct (CI4) and the second end of the fourth hydraulic duct (CI4).

In this case, the electronic control and command device (PLC) is further configured for, in the activating operation d) of the at least one from between the first heat-sealing element (TS1) and the second heat-sealing element (TS2), which is activatable, activating at least one from between the first heat-sealing (TS1) and the second heat-sealing element (TS2): heating the at least one from between the first heat-sealing element (TS1) and the second heat-sealing element (TS2) which is activatable; and activating the fourth hydraulic intercept device (EV4) so as to hydraulically move the at least one from between the first heat-sealing element (TS1) and the second heat-sealing element (TS2) which is activatable towards the remaining element of the first heat-sealing element (TS1) and the second heat-sealing element (TS2).

The second end of the fourth hydraulic duct (CI4) preferably comprises a hydraulic piston which is connected to the at least one from between the first heat-sealing element (TS1) and the second heat-sealing element (TS2) which is activatable to hydraulically move the at least one.

When the medical device (4) which is clean, disinfected and dry is available, a preferred embodiment of the treatment and packaging method according to the invention is one wherein the predisposing step C) of the medical device (4) comprises substep C1) of predisposing a clean, disinfected and dry medical device (4); and wherein the first predetermined depression and the third predetermined depression are comprised between -25,000 Pa (-250 mbar) and -35,000 Pa (-350 mbar) and more preferably are about -30,000 Pa (-300 mbar). This is because the depression is sufficient to effectively treat a medical device (4) that is clean, disinfected and dry.

When, however, there is not this availability, an embodiment of the treatment and packaging method according to the invention is preferred wherein the predisposing step C) of the medical device (4) comprises substep C2) of predisposing step C) of a clean, disinfected and wet medical device (4); and wherein the first predetermined depression and the third predetermined depression are comprised between -65,000 Pa (-650 mbar) (and, preferably, - 30,000 Pa), and -85,000 Pa (-850 mbar) (and, more preferably, -75,000 Pa). This is because these depressions are necessary for totally eliminating the water from the medical device(4).

With the aim of being able to actuate substep C2) of predisposing step C) of a clean, disinfected and wet medical device (4) with the treatment and packaging apparatus (1) according to the invention, the apparatus can further comprise (see in particular figure 11):
- a fifth hydraulic duct (CI5) having a first end in hydraulic communication with the outside environment (AA) outside of the treatment and packaging apparatus (1) and a second end;
- a further filter (F2): which is a suitable filter for filtering viruses and bacteria from the air (which preferably has pores of dimensions of equal to or smaller than 0.005 µm and more preferably smaller than 0.003 µm); and which is arranged along the fifth hydraulic duct (CI5) between the first end of the fifth hydraulic duct (CI5) and the second end of the fifth hydraulic duct (CI5) for filtering the gases conveyed along the fifth hydraulic duct (CI5);
- a compressed-air pistol (PA) hydraulically connected to the second end of the fourth duct;
- a further compressor (C): which is in hydraulic connection with the fifth hydraulic duct (CI5) at a fourth connecting point which is arranged along the fifth hydraulic duct (CI5) between the further filter (F2) and the second end of the fifth hydraulic duct (CI5); and which is activatable to convey compressed air between the first end of the fifth hydraulic duct (CI5) and the second end of the fifth hydraulic duct (CI5).

In a preferred embodiment of the treatment and packaging apparatus (1) of the invention, one of at least a pressure sensor (SP1, SP2) is a pair of pressure sensors comprising: an electronic pressure sensor (preferably from 4-20 mA with a detecting range from 0 to -100,000 Pa (-1000 mbar); and a mechanical sensor, preferably of type 0/1 rateable with a detection range from 0 to -100,000 Pa (-1000 mbar).

The supply end (11) of the supply device advantageously comprises (and, preferably, consists of) a tubular element with a rectangular section having a smaller side and a larger side in which the larger side has a length of between 50-60 times, preferably 55-60, the length of the smaller side (see figures 3 and 13-15). This facilitates the supply of the treatment gas into the heat-sealable unit (7). Note that in figures 13-15 the supply end (11) of the supply device has been illustrated with a length that is not in scale with respect to figures 1-9 only for the purpose of clarity.

The compressor and/or the further compressor have a flow-rate that is advantageously comprised between 10 and 200 litres/minute.

In each embodiment of the treatment and packaging apparatus (1) described herein, each hydraulic intercept device (EV1, EV2, EV3, EV4) is advantageously a valve and, still more advantageously, a solenoid.

It is preferable for the first hydraulic intercept device (EV1) to be 1.905 cm (i.e. 3/4 inches) and for the second hydraulic intercept device (EV2) to be 1.27 cm (i.e. 1/2 inch).

The treatment and packaging apparatus (1) can preferably comprise a closure sensor; which is a closure sensor of the housing (2); which is electrically connected to the electronic control and command device which is configured to carry out the above-mentioned operations of treatment and packaging, only following confirmation by the closure sensor of the closure of the housing (2).

It is further preferable for the treatment and packaging apparatus (1) to comprise a plurality of contact elements, which with the housing (2) closed is arranged around the terminal supply end (11) of the supply device. In this case each of the contact elements (14) of the plurality of contact elements is movable from a first blocked position in which it contacts the terminal supply end (11) of the supply device and a second introduction position in which it is distanced from the terminal supply end (11) of the supply device enabling the at least partial introduction of the terminal supply end (11) of the supply device into the distal opening of the heat-sealable bag of the heat-sealable unit (7). In the blocked position, each of the contact elements (14) of the plurality of contact elements contributes to blocking the heat-sealable unit (7) in the operating position, blocking the relative bag (5). Each of the contact elements (14) of the plurality of contact elements preferably comprises an elastically deformable element (preferably a spring) deformable so as to be positionable from the relative blocked position to the introduction position.

The first heat-sealing element (TS1) is preferably the at least one from between the first heat-sealing element (TS1) and the second heat-sealing element (TS2) which is activatable, so that the relative movement is facilitated with respect to the movement of the second heat-sealing element (TS2).

## Claims

1. A treatment and packaging method of a medical device (4), which medical device (4) is invasive and reutilisable and comprises a plurality of internal channels, the treatment and packaging method comprising, in the following order, following steps:
A) predisposing at least an air source which is a source of medical grade air;
B) predisposing a cold plasma source;
C) predisposing a medical device (4): which is a reutilisable invasive medical device; which comprises: an outer surface; a body; a plurality of internal channels, at least partially housed in the body; and a pressure-balance valve; which is arranged at the body and which is configurable in an open configuration so as to place the inside of the body and an environment (AA) outside the body in communication;
wherein the medical device (4) is:
- in a substep C1) of the predisposing step C): clean; disinfected and dry;
- in a substep C2) of the predisposing step C), which is alternative to substep C1): clean; disinfected and wet; and wherein substep C2) of the predisposing step C) comprises at least partially removing the water from the clean, disinfected and wet medical device (4) using medical compressed air;
D) predisposing a bag (5) which is at least a grade for food use: which is sterile;
which is made of a heat-sealable plastic material; which comprises a terminal opening (6); which is able to house internally thereof the medical device (4) predisposed in the predisposing step C) by inserting the medical device through the terminal opening (6);
E) activating the pressure-balance valve to open so as to place the inside of the body of the medical device (4) and an environment (AA) outside of the body in communication, thus obtaining a balanced medical device (4) wherein the inside of the body is at a same pressure as the environment (AA) outside the body;
F) inserting the balanced medical device (4) obtained in the activating step E) internally of the bag arranged in the predisposing step D), obtaining a heat-sealable unit (7) which comprises: the bag (5) and the balanced medical device (4) arranged internally of the bag (5);
G) carrying out at least a treatment cycle, optionally at a predetermined temperature comprised between 20°C and 60°C, wherein each at least a treatment cycle comprises the following substeps:
G1) obtaining, internally of the heat-sealable unit (7), a first predetermined depression;
G2) supplying, to inside the heat-sealable unit (7), a treatment gas comprising: cold plasma; and medical grade air, maintaining, inside the heat-sealable unit (7), a second predetermined depression for a predetermined time interval;
H) obtaining, internally of the heat-sealable unit (7), a third predetermined depression;
I) on conclusion of the obtaining step H), obtaining, internally of the heat-sealable unit (7), a fourth predetermined depression which is lower: than the first predetermined depression; than the second predetermined depression; and than the third predetermined depression; and heat-sealing the bag of the heat-sealable unit (7) obtaining a heat-sealed unit (8) comprising: a heat-sealed bag (9), which is hermetically closed by at least a heat-sealing; and the balanced reutilisable medical device (4), arranged internally of the heat-sealed bag (9), wherein, a fourth predetermined depression is present internally of the heat-sealed bag (9), which is hermetically closed.

2. The treatment and packaging method of the preceding claim, wherein step G) of carrying out the at least a treatment cycle is the following:
G) carrying out at least a treatment cycle, wherein, when substep C2) is included of the predisposing step C), the at least a treatment cycle is carried out at a predetermined temperature comprised between 20°C and 60°C (preferably between 25°C and 45°C more preferably between 35°C and 45°C), and wherein, when substep C1) of step C) of the predisposing step is included, the at least a treatment cycle is optionally carried out at the predetermined temperature, which can preferably be comprised between 25°C and 45°C, and wherein each treatment cycle comprises substeps G1) and G2).

3. The treatment and packaging method of any one preceding claim, wherein the second predetermined depression is lower than the first predetermined depression, wherein the third predetermined depression is greater than the second predetermined depression.

4. The treatment and packaging method of any one preceding claim, wherein the first predetermined depression and the third predetermined depression are comprised between -10,000 Pa and -100,000 Pa and the second predetermined depression is comprised between -10,000 Pa and -25,000 Pa.

5. The treatment and packaging method of the preceding claim, wherein the first predetermined depression and the third predetermined depression are comprised between -25,000 Pa and -75,000 Pa.

6. The treatment and packaging method of any one preceding claim, wherein in the step G) of carrying out at least a treatment cycle, a plurality of disinfection cycles comprised between 2 and 6 is carried out and the predetermined time interval is comprised between 1 and 300 seconds.

7. The treatment and packaging method of any one preceding claim, wherein the predisposing step C) of the medical device (4) comprises substep C1) of predisposing a clean, disinfected and dry medical device (4); and wherein the first predetermined depression and the third predetermined depression are comprised between -25,000 Pa and -35,000 Pa.

8. The treatment and packaging method according to any one of claims from 1 to 6, wherein predisposing step C) of the medical device (4) comprises substep C2) of predisposing step C) of a clean, disinfected and wet medical device (4); and wherein the first predetermined depression and the third predetermined depression are comprised between -65,000 Pa and -85,000 Pa.

9. The treatment and packaging method of any one preceding claim, wherein the fourth depression is comprised between -10,000 Pa and -20,000 Pa.

10. The heat-sealed unit (8) obtainable by carrying out the treatment and packaging method according to the preceding claims, the heat-sealed unit (8) comprising:
- a heat-sealed bag (9); which is made of a heat-sealable plastic material; and which is hermetically closed by at least a heat-sealing (10); and
- a medical device (4), which is a reutilisable invasive medical device; which comprises: a body; a plurality of internal channels at least partially housed in the body; and a pressure-balance valve, which is arranged at the body and which is activatable to open so as to place the inside of the body and an environment (AA) outside the body in communication, wherein the pressure-balance valve is activated to open; and wherein the medical device (4) is dry and is arranged internally of the heat-sealed bag (9), and wherein a predetermined depression is present internally of the heat-sealed bag (9).

11. The heat-sealed unit (8) of the preceding claim, wherein a predetermined depression is present internally of the heat-sealed bag (9), being comprised between -10,000 Pa and -20,000 Pa.

12. A treatment and packaging apparatus (1) of a reutilisable invasive medical device, the disinfection apparatus comprising:
- a housing (2) comprising: an opening, a cover (3) for closing the opening; and a side; wherein the opening comprises a side wherein the cover (3) comprises a side and with the cover (3) closing the opening, the housing (2) is closed, and the side of the opening and the side of the cover (3) are arranged at the side of the housing (2);
- a supply device: which is activatable to supply a gas internally of the housing (2) when housing (2) is closed: the supply device having a terminal supply end (11), wherein the terminal supply end (11): is arranged in the housing (2); and projects from the side of the housing (2);
- a first heat-sealing element (TS1) and a second heat-sealing element (TS2), wherein the first heat-sealing element (TS1) is arranged inside the housing (2) at the side of said housing (2); wherein the second heat-sealing element (TS2) is arranged internally of the housing (2) and is fixed to the cover (3) at the side of the cover (3); wherein, with the housing (2) closed, the first heat-sealing element (TS1) and the second heat-sealing element (TS2) are opposite one another; wherein at least one from between the first heat-sealing element (TS1) and the second heat-sealing element (TS2) is activatable to heat-seal a heat-sealable plastic material, when the heat-sealable plastic material is interposed between, and in contact with, the first heat-sealing element (TS1) and the second heat-sealing element (TS2), which are opposite one another; and wherein the housing (2) is dimensioned so as to house a heat-sealable unit (7) in an operating position, which heat-sealable unit (7) comprises: a bag (5), which is made of the heat-sealable plastic material and which comprises a terminal opening (6) and a sealing section (12) which is proximal to the terminal opening (6); and a medical device (4), which is a reutilisable invasive medical device, which comprises a plurality of internal channels, and which is arranged internally of the bag; wherein, with the heat-sealable unit (7) in the operating position and the housing (2) closed, the bag of the heat-sealable unit (7) has: the terminal opening (6) arranged around, and in contact with, the terminal supply end (11) of the supply device, and the sealing section (12) which is arranged between the first heat-sealing element (TS1) and the second heat-sealing element (TS2), which are opposite one another, in order to enable a hermetic closing by heat-sealing of the bag (5);
- a production device (PL), which is hydraulically connectable to the supply device and which is activatable to produce cold plasma;
- a suction device which is activatable so as to obtain, with the housing (2) closed, a depression internally of the housing (2)
- at least a pressure sensor (SP1, SP2); which is hydraulically connected to the housing (2); and which is activatable to measure a pressure in the housing (2); and
- an electronic control and command device, which is electrically connected:
to the supply device; to the production device (PL); to the suction device; to the at least a pressure sensor (SP1, SP2); and to the at least one from between the first heat-sealing element (TS1) and the second heat-sealing element (TS2) which is activatable; wherein the electronic control and command device (PLC) is configured to activate said at least a pressure sensor (SP1, SP2) and to monitor the pressure internally of the housing (2) by means of the at least an activated pressure sensor (SP1, SP2) and, when the housing (2) is closed, to activate the production device (PL) and to carry out the following treatment and packaging operations in order as follows:
a) disinfecting by carrying out :
a1) activating the suction device so that said at least a pressure sensor (SP1, SP2) measures a first predetermined depression;
a2) hydraulically connecting the production device (PL) to the first supply device, and activating the supply device to supply a treatment gas, comprising medical grade air and cold plasma, into the housing (2), maintaining, internally of the housing (2), a second depression for a predetermined time interval;
b) activating the suction device so that said at least a pressure sensor (SP1, SP2) measures a third predetermined depression;
c) activating the suction device so that said at least a pressure sensor (SP1, SP2) measures a fourth predetermined depression which is lower:
than the first predetermined depression; than the second predetermined depression; and than the third predetermined depression;
d) activating the at least one from between the first heat-sealing element (TS1) and the second heat-sealing element (TS2) which is activatable, maintaining the fourth predetermined depression; and
f) deactivating the suction device, obtaining, in the housing (2), a pressure that is equal to the environment pressure (AA) outside of the treatment and packaging apparatus (1).

13. The treatment and packaging apparatus of the preceding claim, wherein the housing (2) further comprises a bottom wall (13), which, when the cover (3) closes the housing, is opposite the cover (3), wherein the treatment and packaging apparatus further comprises:
- a heating element (R): and which is activatable so as to be heated; and which is arranged externally of the housing (3) at and in contact with the bottom wall (13);
- at least a first temperature sensor (ST1): which is arranged in the housing (2); and which is activatable to measure the temperature internally of the housing (2);
- at least a second temperature sensor (ST2); which is arranged proximally to the heating element (R); and which is activatable to measure the temperature of the heating element (R);
wherein the electronic control and command device (PLC) is further electrically connected: to the heating element (R); to the at least a first temperature sensor (ST1); to the at least a second temperature sensor (ST2); and wherein the electronic control and command device (PLC) is further configured for, before carrying out the treatment and packaging operation a)-f), activating:
the heating element (R); the at least a first temperature sensor; and the at least a second temperature sensor; maintaining the housing at a temperature of 20°C - 60°C internally of the housing (2) by means of the at least a first temperature sensor (ST1) and monitoring the temperature at the heating element (R) by means of the at least a second temperature sensor (ST1).

14. The treatment and packaging apparatus according to any one claim from 12 to 13, further comprising:
- a first hydraulic duct (CI1): having a first end in hydraulic communication with the environment (AA) outside the treatment and packaging apparatus (1) and a second end, wherein the production device (PL) is hydraulically connectable to the first hydraulic duct (C11) at a first connecting point, which is arranged along the first hydraulic duct (C11) between the first end of the first hydraulic duct (C11) and the second end of the first hydraulic duct (CI1);
- a filter (F1): which is a suitable filter for filtering viruses and bacteria from the air; and which is arranged along the first hydraulic duct (C11) between the first connecting point and the second end of the first hydraulic duct;
- a first hydraulic intercept device (EV1) which is arranged along the first hydraulic duct (C11) between the filter (F1) and the second end of the first hydraulic duct (CI1);
- a second hydraulic duct (CI2) which has: a first end (15) which is in hydraulic communication with the housing (2); and a second end which is in hydraulic communication with the environment (AA) outside the treatment and packaging apparatus (1);
- a second hydraulic intercept device (EV2) which is arranged along the second hydraulic duct (cI2) between the first end (15) of the second hydraulic duct (cI2) and the second end of the second hydraulic duct;
- a suction element (P) which is arranged along the second hydraulic duct (cI2) between the second end of the second hydraulic duct (cI2) and the second hydraulic intercept device (EV2);
- a third hydraulic duct (CI3) which: has a first end which is in hydraulic communication at a second connecting point which is arranged along the second hydraulic duct (CI2) between the first end of the second hydraulic duct (cI2) and the second hydraulic intercept device (EV2); and a second end which is in hydraulic communication with the environment (AA) outside the treatment and packaging apparatus (1);
- a third hydraulic intercept device (EV3) arranged along the third hydraulic duct between the first end of the third hydraulic duct (cI3) and the second end of the third hydraulic duct (CI3);
wherein said at least a pressure sensor (SP1, SP2) is arranged in a position selected from between: a first position internally of the housing (2); a second position arranged along the second hydraulic duct between the first end (15) of the second hydraulic duct (CI2) and the second hydraulic intercept device (EV2); a third position arranged along the third hydraulic duct (cI3) between the first end of the third hydraulic duct (CI3) and the third hydraulic intercept device (EV3); and a fourth position arranged along the first hydraulic duct (C11) between the first hydraulic intercept device (EV1) and the second end of the first hydraulic duct;
wherein the suction device comprises: a portion of the first hydraulic duct (C11) arranged between the first hydraulic intercept device (EV1) and the second end of the first hydraulic duct, the first hydraulic intercept device (EV1), the second hydraulic duct (CI2); the second hydraulic intercept device (EV2); the suction device (P); the third hydraulic duct (CI3); the third hydraulic intercept device (EV3);
wherein the supply device comprises: the suction device; the filter (F1); a portion of the first duct comprised between the first hydraulic intercept device (EV1) and the first end of the first hydraulic duct (C11) and wherein the second end of the first hydraulic duct (C11) constitutes the terminal supply end (11) of the supply device;
wherein the second depression is lower than the first predetermined depression, wherein the third predetermined depression is greater than the second predetermined depression; and wherein the fourth depression is lower than the third depression;
wherein the electronic control and command device (PLC) is further electrically connected: to the first hydraulic intercept device (EV1); to the second hydraulic intercept device (EV2); to the third hydraulic intercept device (EV3), to the suction element (P); and wherein the electronic control and command device (PLC) is further configured in order to carry out following treatment and packaging operations a-c), in the following order:
a) disinfecting by carrying out:
a1) activating the suction device, so that said at least a pressure sensor (SP1, SP2) measures the first predetermined depression: activating the first hydraulic intercept device (EV1) to close; activating the third hydraulic intercept device (EV3) to close; activating the second hydraulic intercept device (EV2) to open; and activating the suction element (P);
a2) hydraulically connecting the production device (PL) to the first supply device, and activating the supply device to supply a treatment gas, comprising medical grade air and cold plasma, into the housing (2), maintaining, inside the housing (2), the second depression for a predetermined time interval, activating the first hydraulic intercept device to open (EV1);
b) activating the suction device, so that said at least a pressure sensor (SP1, SP2) measures the third predetermined depression, activating the first hydraulic intercept device to close (EV1);
c) activating the suction device so that said at least a pressure sensor (SP1, SP2) measures the fourth predetermined depression, activating the first hydraulic intercept device to open (EV1); and
d) activating the at least one from between the first heat-sealing element (TS1) and the second heat-sealing element (TS2) which is activatable, maintaining the fourth predetermined depression inside the housing (2); and
f) deactivating the suction device, obtaining, in the housing (2), a pressure that is equal to the pressure of environment (AA) outside the treatment and packaging apparatus (1), activating the third hydraulic intercept device to open (EV3).

15. The treatment and packaging apparatus (1) of any one of claims from 12 to 14, wherein the treatment and packaging apparatus further comprises:
- a fourth hydraulic duct (CI4) in turn comprising: a first end which is in hydraulic connection with the first hydraulic duct (C11) at a third connecting point, arranged along the first hydraulic duct (CI1), between the filter (F1) and the first hydraulic intercept device (EV1); and a second end which is connected to at least one from between the first heat-sealing element (TS1) and the second heat-sealing element (TS2) which is activatable to hydraulically move this latter towards the remaining element between the first heat-sealing element (TS1) and the second heat-sealing element (TS2);
- a fourth hydraulic intercept device (EV4) arranged along the fourth hydraulic duct (CI4) between the first end of the fourth hydraulic duct (CI4) and the second end of the fourth hydraulic duct (CI4);
wherein the electronic control and command device (PLC) is further configured for, in the activating operation d) of the at least one from between the first heat-sealing element (TS1) and the second heat-sealing element (TS2) which is activatable, activating at least one from between the first heat-sealing (TS1) and the second heat-sealing element (TS2) heating the at least one from between the first heat-sealing element (TS1) and the second heat-sealing element (TS2) which is activatable; and activating the fourth hydraulic intercept device (EV4) so as to hydraulically move the at least one from between the first heat-sealing element (TS1) and the second heat-sealing element (TS2) which is activatable towards the remaining element of the first heat-sealing element (TS1) and the second heat-sealing element (TS2).

16. The treatment and packaging apparatus (1) of any one of claims from 12 to 15, further comprising:
- a fifth hydraulic duct (CI5) having a first end in hydraulic communication with the outside environment (AA) outside of the treatment and packaging apparatus (1) and a second end;
- a further filter (F2): which is a suitable filter for filtering viruses and bacteria from the air; and which is arranged along the fifth hydraulic duct (CI5) between the first end of the fifth hydraulic duct (CI5) and the second end of the fifth hydraulic duct (CI5) for filtering the gases conveyed along the fifth hydraulic duct (CI5);
- a compressed-air pistol (PA) hydraulically connected to the second end of the fifth duct (CI5);
- a compressor (C): which is in hydraulic connection with the fifth hydraulic duct (CI5) at a fourth connecting point which is arranged along the fifth hydraulic duct (CI5) between the further filter (F2) and the second end of the fifth hydraulic duct (CI5); and which is activatable to convey compressed air between the first end of the fifth hydraulic duct (CI5) and the second end of the fifth hydraulic duct (CI5).

17. The treatment and packaging apparatus (1) of any one of claims from 12 to 16, further comprising a RFID label reading device, wherein the RFID label reading device is arranged in the housing (2), is electrically connected to the electronic control and command device (PLC) and is activatable to read a RFID label, wherein the first control and command device (PLC) is further configured, before carrying out the treatment and packaging operation, to activate the RFID reading device and, only following confirmation, by the RFID reading device, that a RFID label reading has taken place, to carry out the treatment and packaging operation.

## Patentansprüche

1. Verfahren zur Aufbereitung und Verpackung eines medizinischen Geräts (4), wobei das medizinische Gerät (4) invasiv und wiederverwendbar ist und mehrere interne Kanäle umfasst, wobei das Verfahren zur Aufbereitung und Verpackung in der folgenden Reihenfolge die folgenden Schritte umfasst:
A) Bereitstellen mindestens einer Luftquelle, bei der es sich um eine Quelle für Luft in medizinischer Qualität handelt;
B) Bereitstellen einer Kaltplasmaquelle;
C) Bereitstellen eines medizinischen Geräts (4): bei dem es sich um ein wiederverwendbares invasives medizinisches Gerät handelt; das umfasst: eine Außenfläche; einen Körper; eine Vielzahl von inneren Kanälen, die zumindest teilweise im Körper untergebracht sind; und ein Druckausgleichsventil; das am Körper angeordnet ist und in eine offene Konfiguration gebracht werden kann, um das Innere des Körpers und eine Umgebung (AA) außerhalb des Körpers miteinander in Verbindung zu bringen;
wobei das medizinische Gerät (4):
- in einem Teilschritt C1) des Vorbereitungsschritts C): sauber; desinfiziert und trocken ist;
- in einem Teilschritt C2) des Vorbereitungsschritts C), der alternativ zu Teilschritt C1) ist: sauber; desinfiziert und nass ist; und wobei Teilschritt C2) des Vorbereitungsschritts C) das zumindest teilweise Entfernen des Wassers aus dem sauberen, desinfizierten und nassen medizinischen Gerät (4) unter Verwendung von medizinischer Druckluft umfasst;
D) Vorbereiten eines Beutels (5), der mindestens für Lebensmittel geeignet ist: der steril ist;
der aus einem heißsiegelbaren Kunststoffmaterial besteht; der eine Endöffnung (6) aufweist; der in der Lage ist, das im Vorbereitungsschritt C) vorbereitete medizinische Gerät (4) in seinem Inneren aufzunehmen, indem das medizinische Gerät durch die Endöffnung (6) eingeführt wird;
E) Aktivieren des Druckausgleichsventils, um es zu öffnen, damit das Innere des Körpers des medizinischen Geräts (4) und eine Umgebung (AA) außerhalb des Körpers miteinander in Verbindung gebracht werden, wodurch ein druckausgeglichenes medizinisches Gerät (4) erhalten wird, bei dem das Innere des Körpers denselben Druck aufweist wie die Umgebung (AA) außerhalb des Körpers;
F) Einführen der im Aktivierungsschritt E) erhaltenen druckausgeglichenen medizinischen Vorrichtung (4) in das Innere des im Vorbereitungsschritt D) angeordneten Beutels, wodurch eine heißsiegelbare Einheit (7) erhalten wird, die umfasst: den Beutel (5) und die im Inneren des Beutels (5) angeordnete druckausgeglichene medizinische Vorrichtung (4);
G) Durchführen mindestens eines Behandlungszyklus, optional bei einer vorbestimmten Temperatur zwischen 20 °C und 60 °C, wobei jeder mindestens eine Behandlungszyklus die folgenden Teilschritte umfasst:
G1) Erzeugen eines ersten vorbestimmten Unterdrucks im Inneren der heißsiegelbaren Einheit (7);
G2) Zuführen eines Behandlungsgases, das kaltes Plasma und medizinische Luft umfasst, in das Innere der heißsiegelbaren Einheit (7), wobei im Inneren der heißsiegelbaren Einheit (7) für ein vorbestimmtes Zeitintervall ein zweiter vorbestimmter Unterdruck aufrechterhalten wird;
H) Erzeugen eines dritten vorbestimmten Unterdrucks im Inneren der heißsiegelbaren Einheit (7);
I) nach Abschluss des Erzeugungsschritts H) Erzeugen eines vierten vorbestimmten Unterdrucks im Inneren der heißsiegelbaren Einheit (7), der niedriger ist: als der erste vorbestimmte Unterdruck; als der zweite vorbestimmte Unterdruck; und als der dritte vorbestimmte Unterdruck; und Heißsiegeln des Beutels der heißsiegelbaren Einheit (7), wodurch eine heißgesiegelte Einheit (8) erhalten wird, die umfasst: einen heißgesiegelten Beutel (9), der durch mindestens eine Heißsiegelung hermetisch verschlossen ist; und die ausgewogene wiederverwendbare medizinische Vorrichtung (4), die im Inneren des heißversiegelten Beutels (9) angeordnet ist, wobei im Inneren des heißversiegelten Beutels (9), der hermetisch verschlossen ist, eine vierte vorbestimmte Vertiefung vorhanden ist.

2. Das Behandlungs- und Verpackungsverfahren nach dem vorstehenden Anspruch, wobei Schritt G) der Durchführung des mindestens einen Behandlungszyklus wie folgt ist:
G) Durchführen mindestens eines Behandlungszyklus, wobei, wenn der Teilschritt C2) des Vorbereitungsschritts C) enthalten ist, der mindestens eine Behandlungszyklus bei einer vorbestimmten Temperatur zwischen 20 °C und 60 °C (vorzugsweise zwischen 25 °C und 45 °C, noch bevorzugter zwischen 35 °C und 45 °C) durchgeführt wird, und wobei, wenn der Teilschritt C1) des Schritts C) des Vorbereitungsschritts enthalten ist, der mindestens eine Behandlungszyklus optional bei der vorbestimmten Temperatur durchgeführt wird, die vorzugsweise zwischen 25 °C und 45 °C liegen kann, und wobei jeder Behandlungszyklus die Teilschritte G1) und G2) umfasst.

3. Das Behandlungs- und Verpackungsverfahren nach einem der vorstehenden Ansprüche, wobei der zweite vorbestimmte Unterdruck niedriger ist als der erste vorbestimmte Unterdruck, wobei der dritte vorbestimmte Unterdruck größer ist als der zweite vorbestimmte Unterdruck.

4. Das Behandlungs- und Verpackungsverfahren nach einem der vorstehenden Ansprüche, wobei der erste vorbestimmte Unterdruck und der dritte vorbestimmte Unterdruck zwischen -10.000 Pa und -100.000 Pa liegen und der zweite vorbestimmte Unterdruck zwischen -10.000 Pa und -25.000 Pa liegt.

5. Das Behandlungs- und Verpackungsverfahren nach einem der vorstehenden Ansprüche, wobei der erste vorbestimmte Unterdruck und der dritte vorbestimmte Unterdruck zwischen -25.000 Pa und -75.000 Pa liegen.

6. Das Behandlungs- und Verpackungsverfahren nach einem der vorstehenden Ansprüche, wobei in dem Schritt G) der Durchführung mindestens eines Behandlungszyklus eine Vielzahl von Desinfektionszyklen im Bereich von 2 bis 6 durchgeführt wird und das vorbestimmte Zeitintervall im Bereich von 1 bis 300 Sekunden liegt.

7. Das Behandlungs- und Verpackungsverfahren nach einem der vorstehenden Ansprüche, wobei der Vorbereitungsschritt C) des medizinischen Geräts (4) den Teilschritt C1) der Vorbereitung eines sauberen, desinfizierten und trockenen medizinischen Geräts (4) umfasst; und wobei der erste vorbestimmte Unterdruck und der dritte vorbestimmte Unterdruck zwischen -25.000 Pa und -35.000 Pa liegen.

8. Das Behandlungs- und Verpackungsverfahren gemäß einem der Ansprüche 1 bis 6, wobei der Vorbereitungsschritt C) des medizinischen Geräts (4) den Teilschritt C2) der Vorbereitung eines sauberen, desinfizierten und feuchten medizinischen Geräts (4) umfasst; und wobei der erste vorbestimmte Unterdruck und der dritte vorbestimmte Unterdruck zwischen -65.000 Pa und - 85.000 Pa liegen.

9. Das Behandlungs- und Verpackungsverfahren nach einem der vorstehenden Ansprüche, wobei der vierte Unterdruck zwischen -10.000 Pa und -20.000 Pa liegt.

10. Die heißversiegelte Einheit (8), die durch Durchführung des Behandlungsund Verpackungsverfahrens gemäß den vorstehenden Ansprüchen erhältlich ist, wobei die heißversiegelte Einheit (8) umfasst:
- einen heißversiegelten Beutel (9), der aus einem heißversiegelbaren Kunststoffmaterial besteht und durch mindestens eine Heißversiegelung (10) hermetisch verschlossen ist; und
- ein medizinisches Gerät (4), das ein wiederverwendbares invasives medizinisches Gerät ist; das umfasst: einen Körper; eine Vielzahl von inneren Kanälen, die zumindest teilweise in dem Körper untergebracht sind; und ein Druckausgleichsventil, das an dem Körper angeordnet ist und das zum Öffnen aktiviert werden kann, um das Innere des Körpers und eine Umgebung (AA) außerhalb des Körpers miteinander in Verbindung zu bringen, wobei das Druckausgleichsventil zum Öffnen aktiviert wird; und wobei das medizinische Gerät (4) trocken ist und im Inneren des heißversiegelten Beutels (9) angeordnet ist, und wobei im Inneren des heißversiegelten Beutels (9) ein vorbestimmter Unterdruck herrscht.

11. Die heißversiegelte Einheit (8) nach dem vorstehenden Anspruch, wobei im Inneren des heißversiegelten Beutels (9) ein vorbestimmter Unterdruck vorhanden ist, der zwischen -10.000 Pa und -20.000 Pa liegt.

12. Eine Behandlungs- und Verpackungsvorrichtung (1) für eine wiederverwendbare invasive medizinische Vorrichtung, wobei die Desinfektionsvorrichtung umfasst:
- ein Gehäuse (2), das umfasst: eine Öffnung, eine Abdeckung (3) zum Verschließen der Öffnung; und eine Seite; wobei die Öffnung eine Seite umfasst, wobei die Abdeckung (3) eine Seite umfasst und wenn die Abdeckung (3) die Öffnung verschließt, das Gehäuse (2) geschlossen ist und die Seite der Öffnung und die Seite der Abdeckung (3) an der Seite des Gehäuses (2) angeordnet sind;
- eine Zuführvorrichtung, die betätigbar ist, um ein Gas in das Innere des Gehäuses (2) zuzuführen, wenn das Gehäuse (2) geschlossen ist, wobei die Zuführvorrichtung ein endseitiges Zuführende (11) aufweist, wobei das endseitige Zuführende (11): im Gehäuse (2) angeordnet ist; und aus der Seite des Gehäuses (2) herausragt;
- ein erstes Heißsiegelelement (TS1) und ein zweites Heißsiegelelement (TS2), wobei das erste Heißsiegelelement (TS1) im Inneren des Gehäuses (2) an der Seite des Gehäuses (2) angeordnet ist; wobei das zweite Heißsiegelelement (TS2) im Inneren des Gehäuses (2) angeordnet und an der Seite des Deckels (3) am Deckel (3) befestigt ist ; wobei bei geschlossenem Gehäuse (2) das erste Heißsiegelelement (TS1) und das zweite Heißsiegelelement (TS2) einander gegenüberliegen; wobei mindestens eines der ersten Heißsiegelelemente (TS1) und des zweiten Heißsiegelelements (TS2) aktivierbar ist, um ein heißsiegelbares Kunststoffmaterial heißzuversiegeln, wenn das heißsiegelbare Kunststoffmaterial zwischen dem ersten Heißsiegelelement (TS1) und dem zweiten Heißsiegelelement (TS2), die einander gegenüberliegen, angeordnet ist und mit diesen in Kontakt steht; und wobei das Gehäuse (2) so dimensioniert ist, dass es eine heißsiegelbare Einheit (7) in einer Betriebsposition aufnimmt, wobei die heißsiegelbare Einheit (7) umfasst: einen Beutel (5), der aus dem heißsiegelbaren Kunststoffmaterial besteht und eine Endöffnung (6) sowie einen Siegelabschnitt (12) aufweist, der sich in der Nähe der Endöffnung (6) liegt; und eine medizinische Vorrichtung (4), bei der es sich um eine wiederverwendbare invasive medizinische Vorrichtung handelt, die eine Vielzahl von inneren Kanälen umfasst und die im Inneren des Beutels angeordnet ist; wobei, wenn sich die heißsiegelbare Einheit (7) in der Betriebsposition befindet und das Gehäuse (2) geschlossen ist, der Beutel der heißsiegelbaren Einheit (7) Folgendes aufweist: die Anschlussöffnung (6), die um das Zuführungsende (11) der Zuführvorrichtung herum angeordnet ist und mit diesem in Kontakt steht, sowie den Dichtungsabschnitt (12), der zwischen dem ersten Heißsiegelelement (TS1) und dem zweiten Heißsiegelelement (TS2) angeordnet ist, die einander gegenüberliegen, um einen hermetischen Verschluss des Beutels durch Heißsiegeln zu ermöglichen (5);
- eine Erzeugungsvorrichtung (PL), die hydraulisch mit der Zuführvorrichtung verbindbar ist und zur Erzeugung von kaltem Plasma betätigbar ist;
- eine Saugvorrichtung, die betätigbar ist, um bei geschlossenem Gehäuse (2) einen Unterdruck im Inneren des Gehäuses (2) zu erzeugen;
- mindestens einen Drucksensor (SP1, SP2), der hydraulisch mit dem Gehäuse (2) verbunden ist und der zur Messung eines Drucks im Gehäuse (2) aktivierbar ist; und
- eine elektronische Steuer- und Steuervorrichtung, die elektrisch verbunden ist:
mit der Zuführvorrichtung; mit der Erzeugungsvorrichtung (PL); mit der Absaugvorrichtung; mit dem mindestens einen Drucksensor (SP1, SP2); und mit dem mindestens einen der ersten Heißsiegelvorrichtung (TS1) und der zweiten Heißsiegelvorrichtung (TS2), die aktivierbar ist; wobei die elektronische Steuer- und Steuervorrichtung (PLC) so konfiguriert ist, dass sie den mindestens einen Drucksensor (SP1, SP2) aktiviert und den Druck im Inneren des Gehäuses (2) mittels des mindestens einen aktivierten Drucksensors (SP1, SP2) überwacht und, wenn das Gehäuse (2) geschlossen ist, die Produktionsvorrichtung (PL) aktiviert und die folgenden Behandlungsund Verpackungsvorgänge in der folgenden Reihenfolge durchführt:
a) Desinfizieren durch Durchführung von:
a1) Aktivieren der Absaugvorrichtung, so dass der mindestens eine Drucksensor (SP1, SP2) einen ersten vorbestimmten Unterdruck misst;
a2) hydraulisches Verbinden der Produktionsvorrichtung (PL) mit der ersten Zufuhrvorrichtung und Aktivieren der Zufuhrvorrichtung, um ein Behandlungsgas, bestehend aus medizinischer Luft und kaltem Plasma, in das Gehäuse (2) zuzuführen, wobei im Inneren des Gehäuses (2) für ein vorbestimmtes Zeitintervall ein zweiter Unterdruck aufrechterhalten wird;
b) Aktivieren der Absaugvorrichtung, so dass der mindestens eine Drucksensor (SP1, SP2) einen dritten vorbestimmten Unterdruck misst;
c) Aktivieren der Absaugvorrichtung, so dass der mindestens eine Drucksensor (SP1, SP2) einen vierten vorbestimmten Unterdruck misst, der niedriger ist:
als der erste vorbestimmte Unterdruck; als der zweite vorbestimmte Unterdruck; und als der dritte vorbestimmte Unterdruck;
d) Aktivieren des mindestens einen der ersten Heißsiegelelemente (TS1) und der zweiten Heißsiegelelemente (TS2), das aktivierbar ist, unter Aufrechterhaltung des vierten vorbestimmten Unterdrucks; und
f) Deaktivieren der Saugvorrichtung, wodurch im Gehäuse (2) ein Druck erreicht wird, der dem Umgebungsdruck (AA) außerhalb der Behandlungsund Verpackungsvorrichtung (1).

13. Die Behandlungs- und Verpackungsvorrichtung nach dem vorstehenden Anspruch, wobei das Gehäuse (2) ferner eine Bodenwand (13) umfasst, die, wenn der Deckel (3) das Gehäuse verschließt, dem Deckel (3) gegenüberliegt, wobei die Behandlungs- und Verpackungsvorrichtung ferner umfasst:
- ein Heizelement (R): das aktivierbar ist, um erwärmt zu werden; und das außerhalb des Gehäuses (3) an der Bodenwand (13) und in Kontakt mit dieser angeordnet ist;
- mindestens einen ersten Temperatursensor (ST1): der im Gehäuse (2) angeordnet ist; und der aktivierbar ist, um die Temperatur im Inneren des Gehäuses (2) zu messen;
- mindestens einen zweiten Temperatursensor (ST2), der in der Nähe des Heizelements (R) angeordnet ist und der zur Messung der Temperatur des Heizelements (R) aktiviert werden kann;
wobei die elektronische Steuer- und Regelvorrichtung (PLC) ferner elektrisch verbunden ist: mit dem Heizelement (R); mit dem mindestens einen ersten Temperatursensor (ST1); mit dem mindestens einen zweiten Temperatursensor (ST2); und wobei die elektronische Steuer- und Regelvorrichtung (PLC) ferner so konfiguriert ist, dass sie vor der Durchführung des Behandlungs- und Verpackungsvorgangs a)-f) Folgendes aktiviert:
das Heizelement (R); den mindestens einen ersten Temperatursensor; und den mindestens einen zweiten Temperatursensor; das Gehäuse mittels des mindestens einen ersten Temperatursensors (ST1) auf einer Temperatur von 20 °C bis 60 °C im Inneren des Gehäuses (2) zu halten und die Temperatur am Heizelement (R) mittels des mindestens einen zweiten Temperatursensors (ST1) zu überwachen.

14. Die Behandlungs- und Verpackungsvorrichtung gemäß einem der Ansprüche 12 bis 13, ferner umfassend:
- eine erste Hydraulikleitung (CI1) : mit einem ersten Ende, das in hydraulischer Verbindung mit der Umgebung (AA) außerhalb der Behandlungs- und Verpackungsvorrichtung (1) steht, und einem zweiten Ende, wobei die Produktionsvorrichtung (PL) an einem ersten Verbindungspunkt, der entlang der ersten Hydraulikleitung (CI1) zwischen dem ersten Ende der ersten Hydraulikleitung (CI1) und dem zweiten Ende der ersten Hydraulikleitung (CI1) angeordnet ist, hydraulisch mit der ersten Hydraulikleitung (CI1) verbindbar ist;
- einen Filter (F1): der ein geeigneter Filter zum Herausfiltern von Viren und Bakterien aus der Luft ist; und der entlang der ersten Hydraulikleitung (CI1) zwischen dem ersten Verbindungspunkt und dem zweiten Ende der ersten Hydraulikleitung angeordnet ist;
- eine erste Hydraulik-Absperrvorrichtung (EV1), die entlang der ersten Hydraulikleitung (CI1) zwischen dem Filter (F1) und dem zweiten Ende der ersten Hydraulikleitung (CI1) angeordnet ist;
- eine zweite Hydraulikleitung (CI2), die aufweist: ein erstes Ende (15), das in hydraulischer Verbindung mit dem Gehäuse (2) steht; und ein zweites Ende, das in hydraulischer Verbindung mit der Umgebung (AA) außerhalb der Behandlungs- und Verpackungsvorrichtung (1) steht;
- eine zweite hydraulische Absperrvorrichtung (EV2), die entlang der zweiten Hydraulikleitung (CI2) zwischen dem ersten Ende (15) der zweiten Hydraulikleitung (CI2) und dem zweiten Ende der zweiten Hydraulikleitung angeordnet ist;
- ein Ansaugelement (P), das entlang der zweiten Hydraulikleitung (CI2) zwischen dem zweiten Ende der zweiten Hydraulikleitung (CI2) und der zweiten hydraulischen Absperrvorrichtung (EV2) angeordnet ist;
- eine dritte Hydraulikleitung (CI3), die: ein erstes Ende aufweist, das an einem zweiten Verbindungspunkt, der entlang der zweiten Hydraulikleitung (CI2) zwischen dem ersten Ende der zweiten Hydraulikleitung (CI2) und der zweiten hydraulischen Absperrvorrichtung (EV2) angeordnet ist, in hydraulischer Verbindung steht; und ein zweites Ende aufweist, das mit der Umgebung (AA) außerhalb der Aufbereitungs- und Verpackungsvorrichtung (1) in hydraulischer Verbindung steht;
- eine dritte hydraulische Absperrvorrichtung (EV3), die entlang der dritten Hydraulikleitung zwischen dem ersten Ende der dritten Hydraulikleitung (CI3) und dem zweiten Ende der dritten Hydraulikleitung (CI3) angeordnet ist;
wobei der mindestens eine Drucksensor (SP1, SP2) an einer Position angeordnet ist, die ausgewählt ist aus: einer ersten Position innerhalb des Gehäuses (2); einer zweiten Position, die entlang der zweiten Hydraulikleitung zwischen dem ersten Ende (15) der zweiten Hydraulikleitung (CI2) und der zweiten hydraulischen Absperrvorrichtung (EV2) angeordnet ist; einer dritten Position, die entlang der dritten Hydraulikleitung (CI3) zwischen dem ersten Ende der dritten Hydraulikleitung (CI3) und der dritten hydraulischen Absperrvorrichtung (EV3) angeordnet ist; und einer vierten Position, die entlang des ersten Hydraulikkanals (CI1) zwischen der ersten hydraulischen Absperrvorrichtung (EV1) und dem zweiten Ende des ersten Hydraulikkanals angeordnet ist;
wobei die Ansaugvorrichtung umfasst: einen Abschnitt des ersten Hydraulikkanals (CI1), der zwischen der ersten hydraulischen Absperrvorrichtung (EV1) und dem zweiten Ende des ersten Hydraulikkanals angeordnet ist, die erste hydraulische Absperrvorrichtung (EV1), den zweiten Hydraulikkanal (CI2); die zweite hydraulische Absperrvorrichtung (EV2); die Ansaugvorrichtung (P); die dritte Hydraulikleitung (CI3); die dritte hydraulische Absperrvorrichtung (EV3);
wobei die Zuführvorrichtung umfasst: die Ansaugvorrichtung; den Filter (F1); einen Abschnitt der ersten Leitung, der zwischen der ersten hydraulischen Absperrvorrichtung (EV1) und dem ersten Ende der ersten Hydraulikleitung (CI1) liegt, und wobei das zweite Ende der ersten Hydraulikleitung (CI1) das Endzuführungsende (11) der Zuführvorrichtung bildet;
wobei der zweite Unterdruck niedriger ist als der erste vorbestimmte Unterdruck, wobei der dritte vorbestimmte Unterdruck größer ist als der zweite vorbestimmte Unterdruck; und wobei der vierte Unterdruck niedriger ist als der dritte Unterdruck;
wobei die elektronische Steuer- und Befehlsvorrichtung (PLC) ferner elektrisch verbunden ist: mit der ersten hydraulischen Absperrvorrichtung (EV1); mit der zweiten hydraulischen Absperrvorrichtung (EV2); mit der dritten hydraulischen Absperrvorrichtung (EV3), mit dem Ansaugelement (P); und wobei die elektronische Steuer- und Befehlsvorrichtung (PLC) ferner so konfiguriert ist, dass sie die folgenden Behandlungs- und Verpackungsvorgänge a-c) in der folgenden Reihenfolge ausführt:
a) Desinfizieren durch Ausführen von:
a1) Aktivieren der Absaugvorrichtung, so dass der mindestens eine Drucksensor (SP1, SP2) den ersten vorbestimmten Unterdruck misst; Aktivieren der ersten hydraulischen Absperrvorrichtung (EV1) zum Schließen; Aktivieren der dritten hydraulischen Absperrvorrichtung (EV3) zum Schließen; Aktivieren der zweiten hydraulischen Absperrvorrichtung (EV2) zum Öffnen; und Aktivieren des Absaugelements (P);
a2) hydraulisches Verbinden der Produktionsvorrichtung (PL) mit der ersten Zufuhrvorrichtung und Aktivieren der Zufuhrvorrichtung, um ein Behandlungsgas, bestehend aus medizinischer Luft und kaltem Plasma, in das Gehäuse (2) zuzuführen, Aufrechterhalten des zweiten Unterdrucks im Inneren des Gehäuses (2) für ein vorbestimmtes Zeitintervall, Aktivieren der ersten hydraulischen Absperrvorrichtung zum Öffnen (EV1);
b) Aktivieren der Absaugvorrichtung, so dass der mindestens eine Drucksensor (SP1, SP2) den dritten vorbestimmten Unterdruck misst, Aktivieren der ersten hydraulischen Absperrvorrichtung zum Schließen (EV1);
c) Aktivieren der Absaugvorrichtung, so dass der mindestens eine Drucksensor (SP1, SP2) den vierten vorbestimmten Unterdruck misst, Aktivieren der ersten hydraulischen Absperrvorrichtung zum Öffnen (EV1); und
d) Aktivieren des mindestens einen der ersten Heißsiegelvorrichtung (TS1) und der zweiten Heißsiegelvorrichtung (TS2), die aktivierbar ist, wobei der vierte vorbestimmte Unterdruck im Inneren des Gehäuses (2) aufrechterhalten wird; und
f) Deaktivieren der Saugvorrichtung, Erreichen eines Drucks im Gehäuse (2), der dem Umgebungsdruck (AA) außerhalb der Behandlungs- und Verpackungsvorrichtung (1) entspricht, sowie Aktivieren der dritten hydraulischen Absperrvorrichtung zum Öffnen (EV3).

15. Die Behandlungs- und Verpackungsvorrichtung (1) nach einem der Ansprüche 12 bis 14, wobei die Behandlungs- und Verpackungsvorrichtung ferner umfasst:
- eine vierte Hydraulikleitung (CI4), die ihrerseits umfasst: ein erstes Ende, das an einem dritten Verbindungspunkt, der entlang der ersten Hydraulikleitung (C11) zwischen dem Filter (F1) und der ersten hydraulischen Absperrvorrichtung (EV1) angeordnet ist, in hydraulischer Verbindung mit der ersten Hydraulikleitung (CI1) steht; und ein zweites Ende, das mit mindestens einem der Elemente aus der Gruppe bestehend aus dem ersten Heißsiegelelement (TS1) und dem zweiten Heißsiegelelement (TS2) verbunden ist, wobei diese Vorrichtung betätigbar ist, um das letztgenannte Element hydraulisch in Richtung des verbleibenden Elements zwischen dem ersten Heißsiegelelement (TS1) und dem zweiten Heißsiegelelement (TS2) zu bewegen;
- eine vierte hydraulische Absperrvorrichtung (EV4), die entlang der vierten Hydraulikleitung (C14) zwischen dem ersten Ende des vierten Hydraulikkanals (C14) und dem zweiten Ende des vierten Hydraulikkanals (C14) angeordnet ist;
wobei die elektronische Steuer- und Befehlsvorrichtung (PLC) ferner so konfiguriert ist, dass sie im Aktivierungsvorgang d) des mindestens einen der ersten Heißsiegelvorrichtung (TS1) und der zweiten Heißsiegelvorrichtung (TS2), die aktivierbar ist, das aktivierbare Element aus dem Bereich zwischen dem ersten Heißsiegelelement (TS1) und dem zweiten Heißsiegelelement (TS2) zu aktivieren; und Aktivieren der vierten hydraulischen Abfangvorrichtung (EV4), um das mindestens eine der zwischen dem ersten Heißsiegelelement (TS1) und dem zweiten Heißsiegelelement (TS2) aktivierbaren Elemente hydraulisch in Richtung des verbleibenden Elements aus dem ersten Heißsiegelelement (TS1) und dem zweiten Heißsiegelelement (TS2) zu bewegen.

16. Die Behandlungs- und Verpackungsvorrichtung (1) nach einem der Ansprüche 12 bis 15, ferner umfassend:
- einen fünften Hydraulikkanal (CI5) mit einem ersten Ende, das in hydraulischer Verbindung mit der Außenumgebung (AA) außerhalb der Behandlungs- und Verpackungsvorrichtung (1) und ein zweites Ende;
- einen weiteren Filter (F2): der ein geeigneter Filter zum Herausfiltern von Viren und Bakterien aus der Luft ist; und der entlang der fünften Hydraulikleitung (CI5) zwischen dem ersten Ende der fünften Hydraulikleitung (CI5) und dem zweiten Ende der fünften Hydraulikleitung (CI5) angeordnet ist, um die entlang der fünften Hydraulikleitung (CI5) geförderten Gase zu filtern;
- eine Druckluftpistole (PA), die hydraulisch mit dem zweiten Ende der fünften Leitung (CI5) verbunden ist;
- einen Kompressor (C): der an einem vierten Verbindungspunkt, der entlang der fünften Hydraulikleitung (CI5) zwischen dem weiteren Filter (F2) und dem zweiten Ende der fünften Hydraulikleitung (CI5) angeordnet ist, in hydraulischer Verbindung mit der fünften Hydraulikleitung (CI5) steht; und der betätigbar ist, um Druckluft zwischen dem ersten Ende der fünften Hydraulikleitung (CI5) und dem zweiten Ende der fünften Hydraulikleitung (CI5) zu befördern.

17. Die Behandlungs- und Verpackungsvorrichtung (1) nach einem der Ansprüche 12 bis 16, ferner umfassend eine RFID-Etiketten-Lesevorrichtung, wobei die RFID-Etiketten-Lesevorrichtung im Gehäuse (2) angeordnet ist, elektrisch mit der elektronischen Steuer- und Befehlsvorrichtung (SPS) verbunden ist und betätigbar ist, um ein RFID-Etikett zu lesen, wobei die erste Steuer- und Befehlsvorrichtung (SPS) ferner so konfiguriert ist, dass sie vor der Durchführung des Behandlungs- und Verpackungsvorgangs die RFID-Lesevorrichtung aktiviert und erst nach Bestätigung durch die RFID-Lesevorrichtung, dass ein RFID-Etikett gelesen wurde, den Behandlungs- und Verpackungsvorgang durchführt.

## Revendications

1. Procédé de traitement et de conditionnement d'un dispositif médical (4), ledit dispositif médical (4) étant invasif et réutilisable et comprenant une pluralité de canaux internes, le procédé de traitement et de conditionnement comprenant, dans l'ordre suivant, les étapes suivantes:
A) mettre en place au moins une source d'air qui est une source d'air de qualité médicale;
B) mettre en place une source de plasma froid;
C) la mise en place d'un dispositif médical (4): qui est un dispositif médical invasif réutilisable ; qui comprend : une surface extérieure ; un corps ; une pluralité de canaux internes, au moins partiellement logés dans le corps ; et une valve d'équilibrage de pression ; qui est disposée sur le corps et qui peut être configurée dans une configuration ouverte de manière à mettre en communication l'intérieur du corps et un environnement (AA) extérieur au corps;
dans lequel le dispositif médical (4) est:
- dans une sous-étape C1) de l'étape de préparation C) : propre ; désinfecté et sec ;
- dans une sous-étape C2) de l'étape de préparation C), qui est alternative à la sous-étape C1): propre; désinfecté et humide; et dans lequel la sous-étape C2) de l'étape de préparation C) comprend l'élimination au moins partielle de l'eau du dispositif médical (4) propre, désinfecté et humide à l'aide d'air comprimé médical;
D) préparer un sac (5) qui est au moins de qualité alimentaire: qui est stérile;
qui est constitué d'un matériau plastique thermoscellable ; qui comprend une ouverture terminale (6); qui est capable de loger à l'intérieur le dispositif médical (4) préparé lors de l'étape de préparation C) en insérant le dispositif médical à travers l'ouverture terminale (6) ;
E) activer la valve d'équilibrage de pression pour l'ouvrir de manière à mettre en communication l'intérieur du corps du dispositif médical (4) et un environnement (AA) extérieur au corps, obtenant ainsi un dispositif médical équilibré (4) dans lequel l'intérieur du corps est à la même pression que l'environnement (AA) extérieur au corps;
F) insérer le dispositif médical équilibré (4) obtenu à l'étape d'activation E) à l'intérieur du sachet disposé à l'étape de prédisposition D), obtenant ainsi une unité thermoscellable (7) qui comprend: le sachet (5) et le dispositif médical équilibré (4) disposé à l'intérieur du sachet (5);
G) effectuer au moins un cycle de traitement, éventuellement à une température prédéterminée comprise entre 20 °C et 60 °C, dans lequel chaque cycle de traitement comprend les sous-étapes suivantes:
G1) obtenir, à l'intérieur de l'unité thermoscellable (7), une première dépression prédéterminée;
G2) fournir, à l'intérieur de l'unité thermoscellable (7), un gaz de traitement comprenant : du plasma froid ; et de l'air de qualité médicale, en maintenant, à l'intérieur de l'unité thermoscellable (7), une deuxième dépression prédéterminée pendant un intervalle de temps prédéterminé;
H) obtenir, à l'intérieur de l'unité thermoscellable (7), une troisième dépression prédéterminée;
I) à l'issue de l'étape d'obtention H), obtenir, à l'intérieur de l'unité thermoscellable (7), une quatrième dépression prédéterminée qui est inférieure : à la première dépression prédéterminée; que la deuxième dépression prédéterminée ; et que la troisième dépression prédéterminée; et thermosceller le sachet de l'unité thermoscellable (7) pour obtenir une unité thermoscellée (8) comprenant : un sachet thermoscellé (9), qui est fermé hermétiquement par au moins un thermoscellage; et le dispositif médical réutilisable équilibré (4), disposé à l'intérieur du sachet thermoscellé (9), dans lequel une quatrième dépression prédéterminée est présente à l'intérieur du sachet thermoscellé (9), qui est hermétiquement fermé.

2. Procédé de traitement et de conditionnement selon la revendication précédente, dans lequel l'étape G) consistant à effectuer au moins un cycle de traitement est la suivante:
G) réaliser au moins un cycle de traitement, dans lequel, lorsque la sous-étape C2) est incluse dans l'étape de prédisposition C), ledit au moins un cycle de traitement est réalisé à une température prédéterminée comprise entre 20 °C et 60 °C (de préférence entre 25 °C et 45 °C, plus préférablement entre 35 °C et 45 °C), et dans lequel, lorsque la sous-étape C1) de l'étape C) de l'étape de prédisposition est incluse, ledit au moins un cycle de traitement est éventuellement effectué à la température prédéterminée, qui peut de préférence être comprise entre 25 °C et 45 °C, et dans lequel chaque cycle de traitement comprend les sous-étapes G1) et G2).

3. Procédé de traitement et de conditionnement selon l'une quelconque des revendications précédentes, dans lequel la deuxième dépression prédéterminée est inférieure à la première dépression prédéterminée, et dans lequel la troisième dépression prédéterminée est supérieure à la deuxième dépression prédéterminée.

4. Procédé de traitement et de conditionnement selon l'une quelconque des revendications précédentes, dans lequel la première dépression prédéterminée et la troisième dépression prédéterminée sont comprises entre -10 000 Pa et -100 000 Pa et la deuxième dépression prédéterminée est comprise entre -10 000 Pa et -25 000 Pa.

5. Procédé de traitement et de conditionnement selon l'une quelconque des revendications précédentes, dans lequel la première dépression prédéterminée et la troisième dépression prédéterminée sont comprises entre -25 000 Pa et -75 000 Pa.

6. Procédé de traitement et de conditionnement selon l'une quelconque des revendications précédentes, dans lequel, à l'étape G) consistant à effectuer au moins un cycle de traitement, une pluralité de cycles de désinfection compris entre 2 et 6 est effectuée et l'intervalle de temps prédéterminé est compris entre 1 et 300 secondes.

7. Procédé de traitement et de conditionnement selon l'une quelconque des revendications précédentes, dans lequel l'étape de préparation C) du dispositif médical (4) comprend la sous-étape C1) consistant à préparer un dispositif médical (4) propre, désinfecté et sec ; et dans lequel la première dépression prédéterminée et la troisième dépression prédéterminée sont comprises entre -25 000 Pa et -35 000 Pa.

8. Procédé de traitement et de conditionnement selon l'une quelconque des revendications 1 à 6, dans lequel l'étape de préparation C) du dispositif médical (4) comprend la sous-étape C2) consistant à préparer un dispositif médical (4) propre, désinfecté et humide ; et dans lequel la première dépression prédéterminée et la troisième dépression prédéterminée sont comprises entre -65 000 Pa et -85 000 Pa.

9. Procédé de traitement et d'emballage selon l'une quelconque des revendications précédentes, dans lequel la quatrième dépression est comprise entre -10 000 Pa et -20 000 Pa.

10. L'unité thermoscellée (8) pouvant être obtenue en mettant en œuvre le procédé de traitement et de conditionnement selon les revendications précédentes, l'unité thermoscellée (8) comprenant :
- un sachet thermoscellé (9) ; qui est fabriqué à partir d'un matériau plastique thermoscellable; et qui est fermé hermétiquement par au moins un thermoscellage (10); et
- un dispositif médical (4), qui est un dispositif médical invasif réutilisable; qui comprend: un corps; une pluralité de canaux internes logés au moins partiellement dans le corps ; et une valve d'équilibrage de pression, qui est disposée sur le corps et qui peut être activée pour s'ouvrir de manière à mettre en communication l'intérieur du corps et un environnement (AA) extérieur au corps, dans lequel la valve d'équilibrage de pression est activée pour s'ouvrir ; et dans lequel le dispositif médical (4) est sec et est disposé à l'intérieur du sachet thermoscellé (9), et dans lequel une dépression prédéterminée est présente à l'intérieur du sachet thermoscellé (9).

11. L'unité thermosoudée (8) selon la revendication précédente, dans laquelle une dépression prédéterminée est présente à l'intérieur du sac thermosoudé (9), comprise entre -10 000 Pa et -20 000 Pa.

12. Appareil de traitement et d'emballage (1) d'un dispositif médical invasif réutilisable, l'appareil de désinfection comprenant :
- un boîtier (2) comprenant: une ouverture, un couvercle (3) destiné à fermer l'ouverture; et un côté ; dans lequel l'ouverture comprend un côté, le couvercle (3) comprend un côté et, lorsque le couvercle (3) ferme l'ouverture, le boîtier (2) est fermé, et le côté de l'ouverture et le côté du couvercle (3) sont disposés sur le côté du boîtier (2);
- un dispositif d'alimentation: qui peut être activé pour fournir un gaz à l'intérieur du boîtier (2) lorsque le boîtier (2) est fermé: le dispositif d'alimentation comportant une extrémité d'alimentation terminale (11), dans lequel l'extrémité d'alimentation terminale (11): est disposée dans le boîtier (2); et fait saillie depuis le côté du boîtier (2);
- un premier élément de thermoscellage (TS1) et un deuxième élément de thermoscellage (TS2), le premier élément de thermoscellage (TS1) étant disposé à l'intérieur du boîtier (2) sur le côté dudit boîtier (2) ; le deuxième élément de thermoscellage (TS2) étant disposé à l'intérieur du boîtier (2) et fixé au couvercle (3) sur le côté du couvercle (3) ; dans lequel, lorsque le boîtier (2) est fermé, le premier élément de thermoscellage (TS1) et le deuxième élément de thermoscellage (TS2) sont en vis-à-vis ; dans lequel au moins l'un parmi le premier élément de thermoscellage (TS1) et le deuxième élément de thermoscellage (TS2) peut être activé pour thermosceller une matière plastique thermoscellable, lorsque la matière plastique thermoscellable est intercalée entre, et en contact avec, le premier élément de thermoscellage (TS1) et le deuxième élément de thermoscellage (TS2), qui sont opposés l'un à l'autre ; et dans lequel le boîtier (2) est dimensionné de manière à loger une unité thermosoudable (7) dans une position de fonctionnement, laquelle unité thermosoudable (7) comprend: un sac (5), qui est constitué de la matière plastique thermosoudable et qui comprend une ouverture terminale (6) et une section de soudure (12) qui est proximale à l'ouverture terminale (6); et un dispositif médical (4), qui est un dispositif médical invasif réutilisable, qui comprend une pluralité de canaux internes, et qui est disposé à l'intérieur du sac; dans lequel, lorsque l'unité thermosoudable (7) est en position de fonctionnement et que le boîtier (2) est fermé, le sac de l'unité thermosoudable (7) présente: l'ouverture terminale (6) disposée autour de l'extrémité d'alimentation terminale (11) du dispositif d'alimentation et en contact avec celle-ci, et la section de scellage (12) qui est disposée entre le premier élément de thermoscellage (TS1) et le deuxième élément de thermoscellage (TS2), qui sont opposés l'un à l'autre, afin de permettre une fermeture hermétique par thermoscellage du sac (5) ;
- un dispositif de production (PL), qui peut être raccordé hydrauliquement au dispositif d'alimentation et qui peut être activé pour produire du plasma froid ;
- un dispositif d'aspiration qui peut être activé de manière à obtenir, lorsque le boîtier (2) est fermé, une dépression à l'intérieur du boîtier (2) ;
- au moins un capteur de pression (SP1, SP2) ; qui est relié hydrauliquement au boîtier (2) ; et qui peut être activé pour mesurer une pression à l'intérieur du boîtier (2) ; et
- un dispositif électronique de commande et de contrôle, qui est relié électriquement :
au dispositif d'alimentation; au dispositif de production (PL); au dispositif d'aspiration ; au au moins un capteur de pression (SP1, SP2) ; et à au moins l'un parmi le premier élément de thermoscellage (TS1) et le deuxième élément de thermoscellage (TS2) qui est activable; dans lequel le dispositif électronique de commande et de contrôle (PLC) est configuré pour activer ledit au moins un capteur de pression (SP1, SP2) et pour surveiller la pression à l'intérieur du boîtier (2) au moyen dudit au moins un capteur de pression activé (SP1, SP2) et, lorsque le boîtier (2) est fermé, pour activer le dispositif de production (PL) et pour effectuer les opérations de traitement et d'emballage suivantes dans l'ordre suivant:
a) désinfection en effectuant:
a1) l'activation du dispositif d'aspiration de sorte que ledit au moins un capteur de pression (SP1, SP2) mesure une première dépression prédéterminée ;
a2) raccorder hydrauliquement le dispositif de production (PL) au premier dispositif d'alimentation, et activer le dispositif d'alimentation pour fournir un gaz de traitement, comprenant de l'air de qualité médicale et du plasma froid, dans le boîtier (2), en maintenant, à l'intérieur du boîtier (2), une deuxième dépression pendant un intervalle de temps prédéterminé ;
b) l'activation du dispositif d'aspiration de telle sorte que ledit au moins un capteur de pression (SP1, SP2) mesure une troisième dépression prédéterminée ;
c) l'activation du dispositif d'aspiration de telle sorte que ledit au moins un capteur de pression (SP1, SP2) mesure une quatrième dépression prédéterminée qui est inférieure :
à la première dépression prédéterminée; à la deuxième dépression prédéterminée; et à la troisième dépression prédéterminée ;
d) activer au moins l'un parmi le premier élément de thermoscellage (TS1) et le deuxième élément de thermoscellage (TS2) qui est activable, en maintenant la quatrième dépression prédéterminée ; et
f) désactiver le dispositif d'aspiration, obtenant, dans le boîtier (2), une pression qui est égale à la pression ambiante (AA) à l'extérieur de l'appareil de traitement et d'emballage (1).

13. Appareil de traitement et d'emballage selon la revendication précédente, dans lequel le boîtier (2) comprend en outre une paroi inférieure (13) qui, lorsque le couvercle (3) ferme le boîtier, est opposée au couvercle (3), l'appareil de traitement et d'emballage comprenant en outre :
- un élément chauffant (R) : qui peut être activé de manière à être chauffé ; et qui est disposé à l'extérieur du boîtier (3) au niveau de la paroi inférieure (13) et en contact avec celle-ci ;
- au moins un premier capteur de température (ST1) : qui est disposé dans le boîtier (2) ; et qui peut être activé pour mesurer la température à l'intérieur du boîtier (2) ;
- au moins un deuxième capteur de température (ST2) ; qui est disposé à proximité de l'élément chauffant (R) ; et qui peut être activé pour mesurer la température de l'élément chauffant (R) ;
dans lequel le dispositif électronique de commande et de contrôle (PLC) est en outre connecté électriquement : à l'élément chauffant (R) ; à au moins un premier capteur de température (ST1) ; à au moins un deuxième capteur de température (ST2) ; et dans lequel le dispositif électronique de commande et de contrôle (PLC) est en outre configuré pour, avant d'effectuer l'opération de traitement et d'emballage a)-f), activer :
l'élément chauffant (R) ; au moins un premier capteur de température ; et au moins un deuxième capteur de température ; maintenir le boîtier à une température comprise entre 20 °C et 60 °C à l'intérieur du boîtier (2) au moyen dudit au moins un premier capteur de température (ST1) et surveiller la température au niveau de l'élément chauffant (R) au moyen dudit au moins un deuxième capteur de température (ST1).

14. Appareil de traitement et de conditionnement selon l'une quelconque des revendications 12 à 13, comprenant en outre :
- un premier conduit hydraulique (CI1) : ayant une première extrémité en communication hydraulique avec l'environnement (AA) à l'extérieur de l'appareil de traitement et de conditionnement (1) et une seconde extrémité, dans lequel le dispositif de production (PL) peut être raccordé hydrauliquement au premier conduit hydraulique (CI1) au niveau d'un premier point de raccordement, qui est disposé le long du premier conduit hydraulique (CI1) entre la première extrémité du premier conduit hydraulique (CI1) et la seconde extrémité du premier conduit hydraulique (CI1) ;
- un filtre (F1): qui est un filtre adapté pour filtrer les virus et les bactéries présents dans l'air ; et qui est disposé le long du premier conduit hydraulique (CI1) entre le premier point de raccordement et la deuxième extrémité du premier conduit hydraulique ;
- un premier dispositif d'interception hydraulique (EV1) qui est disposé le long du premier conduit hydraulique (CI1) entre le filtre (F1) et la deuxième extrémité du premier conduit hydraulique (CI1) ;
- un deuxième conduit hydraulique (CI2) qui comporte : une première extrémité (15) qui est en communication hydraulique avec le boîtier (2) ; et une deuxième extrémité qui est en communication hydraulique avec l'environnement (AA) à l'extérieur de l'appareil de traitement et de conditionnement (1);
- un deuxième dispositif d'interception hydraulique (EV2) qui est disposé le long du deuxième conduit hydraulique (CI2) entre la première extrémité (15) du deuxième conduit hydraulique (CI2) et la deuxième extrémité du deuxième conduit hydraulique ;
- un élément d'aspiration (P) qui est disposé le long du deuxième conduit hydraulique (CI2) entre la deuxième extrémité du deuxième conduit hydraulique (CI2) et le deuxième dispositif d'interception hydraulique (EV2) ;
- un troisième conduit hydraulique (CI3) qui : comporte une première extrémité qui est en communication hydraulique au niveau d'un deuxième point de raccordement disposé le long du deuxième conduit hydraulique (CI2) entre la première extrémité du deuxième conduit hydraulique (CI2) et le deuxième dispositif d'interception hydraulique (EV2) ; et une deuxième extrémité qui est en communication hydraulique avec l'environnement (AA) à l'extérieur de l'appareil de traitement et de conditionnement (1) ;
- un troisième dispositif d'interception hydraulique (EV3) disposé le long du troisième conduit hydraulique entre la première extrémité du troisième conduit hydraulique (CI3) et la deuxième extrémité du troisième conduit hydraulique (CI3) ;
dans lequel ledit au moins un capteur de pression (SP1, SP2) est disposé dans une position choisie parmi : une première position à l'intérieur du boîtier (2) ; une deuxième position disposée le long du deuxième conduit hydraulique entre la première extrémité (15) du deuxième conduit hydraulique (CI2) et le deuxième dispositif d'interception hydraulique (EV2) ; une troisième position disposée le long du troisième conduit hydraulique (CI3) entre la première extrémité du troisième conduit hydraulique (CI3) et le troisième dispositif d'interception hydraulique (EV3) ; et une quatrième position située le long du premier conduit hydraulique (CI1) entre le premier dispositif d'interception hydraulique (EV1) et la deuxième extrémité du premier conduit hydraulique ;
dans lequel le dispositif d'aspiration comprend : une partie du premier conduit hydraulique (CI1) située entre le premier dispositif d'interception hydraulique (EV1) et la deuxième extrémité du premier conduit hydraulique, le premier dispositif d'interception hydraulique (EV1), le deuxième conduit hydraulique (CI2) ; le deuxième dispositif d'interception hydraulique (EV2) ; le dispositif d'aspiration (P) ; le troisième conduit hydraulique (CI3) ; le troisième dispositif d'interception hydraulique (EV3) ;
dans lequel le dispositif d'alimentation comprend : le dispositif d'aspiration ; le filtre (F1) ; une partie du premier conduit comprise entre le premier dispositif d'interception hydraulique (EV1) et la première extrémité du premier conduit hydraulique (CI1) et dans lequel la deuxième extrémité du premier conduit hydraulique (CI1) constitue l'extrémité d'alimentation terminale (11) du dispositif d'alimentation;
dans lequel la deuxième dépression est inférieure à la première dépression prédéterminée, dans lequel la troisième dépression prédéterminée est supérieure à la deuxième dépression prédéterminée; et dans lequel la quatrième dépression est inférieure à la troisième dépression ;
dans lequel le dispositif de commande et de contrôle électronique (PLC) est en outre connecté électriquement: au premier dispositif d'interception hydraulique (EV1) ; au deuxième dispositif d'interception hydraulique (EV2); au troisième dispositif d'interception hydraulique (EV3), à l'élément d'aspiration (P) ; et dans lequel le dispositif électronique de commande et de contrôle (PLC) est en outre configuré pour effectuer les opérations de traitement et de conditionnement suivantes a-c), dans l'ordre suivant:
a) désinfecter en effectuant:
a1) l'activation du dispositif d'aspiration, de sorte que ledit au moins un capteur de pression (SP1, SP2) mesure la première dépression prédéterminée ; l'activation du premier dispositif d'interception hydraulique (EV1) pour le fermer; l'activation du troisième dispositif d'interception hydraulique (EV3) pour le fermer; l'activation du deuxième dispositif d'interception hydraulique (EV2) pour l'ouvrir; et l'activation de l'élément d'aspiration (P) ;
a2) raccorder hydrauliquement le dispositif de production (PL) au premier dispositif d'alimentation, et activer le dispositif d'alimentation pour fournir un gaz de traitement, comprenant de l'air de qualité médicale et du plasma froid, dans le boîtier (2), maintenir, à l'intérieur du boîtier (2), la deuxième dépression pendant un intervalle de temps prédéterminé, activer le premier dispositif d'interception hydraulique pour l'ouvrir (EV1);
b) activer le dispositif d'aspiration, de sorte que ledit au moins un capteur de pression (SP1, SP2) mesure la troisième dépression prédéterminée, activer le premier dispositif d'interception hydraulique pour le fermer (EV1);
c) activer le dispositif d'aspiration de sorte que ledit au moins un capteur de pression (SP1, SP2) mesure la quatrième dépression prédéterminée, activer le premier dispositif d'interception hydraulique pour l'ouvrir (EV1) ; et
d) activer au moins l'un parmi le premier élément de thermoscellage (TS1) et le deuxième élément de thermoscellage (TS2) qui est activable, en maintenant la quatrième dépression prédéterminée à l'intérieur du boîtier (2) ; et
f) désactiver le dispositif d'aspiration, obtenir, dans le boîtier (2), une pression égale à la pression ambiante (AA) à l'extérieur de l'appareil de traitement et de conditionnement (1), activer le troisième dispositif d'interception hydraulique pour l'ouvrir (EV3).

15. L'appareil de traitement et de conditionnement (1) selon l'une quelconque des revendications 12 à 14, dans lequel l'appareil de traitement et de conditionnement comprend en outre :
- un quatrième conduit hydraulique (CI4) comprenant à son tour : une première extrémité qui est en communication hydraulique avec le premier conduit hydraulique (CI1) en un troisième point de raccordement, disposé le long du premier conduit hydraulique (CI1), entre le filtre (F1) et le premier dispositif d'interception hydraulique (EV1) ; et une deuxième extrémité qui est reliée à au moins l'un parmi le premier élément de thermoscellage (TS1) et le deuxième élément de thermoscellage (TS2), lequel est activable pour déplacer hydrauliquement ce dernier vers l'élément restant parmi le premier élément de thermoscellage (TS1) et le deuxième élément de thermoscellage (TS2) ;
- un quatrième dispositif d'interception hydraulique (EV4) disposé le long du quatrième conduit hydraulique (CI4) entre la première extrémité du quatrième conduit hydraulique (CI4) et la deuxième extrémité du quatrième conduit hydraulique (CI4) ;
dans lequel le dispositif électronique de commande et de contrôle (PLC) est en outre configuré pour, lors de l'opération d'activation d) du au moins un élément parmi le premier élément de thermoscellage (TS1) et le deuxième élément de thermoscellage (TS2) pouvant être activé, activer au moins l'un parmi le premier élément de thermoscellage (TS1) et le deuxième élément de thermoscellage (TS2) ; chauffer ledit au moins un parmi le premier élément de thermoscellage (TS1) et le deuxième élément de thermoscellage (TS2) qui est activable ; et activer le quatrième dispositif d'interception hydraulique (EV4) de manière à déplacer hydrauliquement l'au moins un parmi le premier élément de thermoscellage (TS1) et le deuxième élément de thermoscellage (TS2) qui est activable vers l'élément restant parmi le premier élément de thermoscellage (TS1) et le deuxième élément de thermoscellage (TS2).

16. L'appareil de traitement et d'emballage (1) selon l'une quelconque des revendications 12 à 15, comprenant en outre :
- un cinquième conduit hydraulique (CI5) ayant une première extrémité en communication hydraulique avec l'environnement extérieur (AA) à l'extérieur de l'appareil de traitement et d'emballage (1) et une seconde extrémité ;
- un filtre supplémentaire (F2) : qui est un filtre adapté pour filtrer les virus et les bactéries présents dans l'air ; et qui est disposé le long du cinquième conduit hydraulique (CI5) entre la première extrémité du cinquième conduit hydraulique (CI5) et la seconde extrémité du cinquième conduit hydraulique (CI5) pour filtrer les gaz acheminés le long du cinquième conduit hydraulique (CI5) ;
- un pistolet à air comprimé (PA) relié hydrauliquement à la deuxième extrémité du cinquième conduit (CI5) ;
- un compresseur (C) : qui est en communication hydraulique avec le cinquième conduit hydraulique (CI5) au niveau d'un quatrième point de raccordement disposé le long du cinquième conduit hydraulique (CI5) entre le filtre supplémentaire (F2) et la deuxième extrémité du cinquième conduit hydraulique (CI5) ; et qui peut être activé pour acheminer de l'air comprimé entre la première extrémité du cinquième conduit hydraulique (CI5) et la deuxième extrémité du cinquième conduit hydraulique (CI5).

17. Appareil de traitement et d'emballage (1) selon l'une quelconque des revendications 12 à 16, comprenant en outre un dispositif de lecture d'étiquettes RFID, dans lequel le dispositif de lecture d'étiquettes RFID est disposé dans le boîtier (2), est connecté électriquement au dispositif électronique de commande et de contrôle (PLC) et peut être activé pour lire une étiquette RFID, le premier dispositif de commande et de contrôle (PLC) étant en outre configuré, avant d'exécuter l'opération de traitement et d'emballage, pour activer le dispositif de lecture RFID et, uniquement après confirmation par le dispositif de lecture RFID qu'une lecture d'étiquette RFID a eu lieu, pour exécuter l'opération de traitement et d'emballage.
